(19) **Europäisches Patentamt**

**European Patent Office**

**Office européen des brevets**

(11) **EP 1 298 222 B1**

(12) **EUROPEAN PATENT SPECIFICATION**

(45) Date of publication and mention
of the grant of the patent:
**11.10.2006 Bulletin 2006/41**

(51) Int Cl.:
***C12Q 1/68*** (2006.01)

(21) Application number: **02019424.7**

(22) Date of filing: **30.08.2002**

(54) **Methods to evaluate rice palatability and to select rice with palatable traits.**

Verfahren zur Abschätzung der Schmackhaftigkeit des Reises und zur Auswahl eines schmackhaften Reises.

Méthodes d'évaluation de la sapidité du riz et de sélection de riz présentant le caractère de sapidité.

(84) Designated Contracting States:
**ES FR IT**

(30) Priority: **10.09.2001 JP 2001273689**

(43) Date of publication of application:
**02.04.2003 Bulletin 2003/14**

(73) Proprietors:
• **National Food Research Institute**
**Tsukuba-shi, Ibaraki 305-8642 (JP)**
• **Incorporated Administrative Agency National**
**Agriculture and Bio-oriented Research**
**Organization**
**Ibaraki (JP)**

(72) Inventors:
• **Ohtsubo, Kenichi**
**Ibaraki (JP)**
• **Okadome, Hiroshi**
**Tsukuba-shi,**
**Ibaraki (JP)**
• **Nakamura, Sumiko**
**Inashiki-gun,**
**Ibaraki (JP)**
• **Haraguchi, Kazutomo**
**Tsukuba-shi,**
**Ibaraki (JP)**
• **Yoza, Kouichi**
**Tsukuba-shi,**
**Ibaraki (JP)**
• **Okunishi, Tomoya**
**Ushiku-shi,**
**Ibaraki (JP)**
• **Suzuki, Keitaro**
**Ushiku-shi,**
**Ibaraki (JP)**

(74) Representative: **Gille Hrabal Struck Neidlein Prop**
**Roos**
**Patentanwälte**
**Brucknerstrasse 20**
**40593 Düsseldorf (DE)**

(56) References cited:
• **LORIEUX M ET AL: "Aroma in rice: Genetic analysis of a quantitative trait." THEORETICAL AND APPLIED GENETICS, vol. 93, no. 7, 1996, pages 1145-1151, XP009003992 ISSN: 0040-5752**
• **LANCERAS J C ET AL: "Mapping of genes for cooking and eating qualities in Thai jasmine rice (KDML105)." DNA RESEARCH: AN INTERNATIONAL JOURNAL FOR RAPID PUBLICATION OF REPORTS ON GENES AND GENOMES. JAPAN 28 APR 2000, vol. 7, no. 2, 28 April 2000 (2000-04-28), pages 93-101, XP002227767 ISSN: 1340-2838**
• **TAN Y F ET AL: "Genetic bases of appearance quality of rice grains in Shanyou 63, an elite rice hybrid." THEORETICAL AND APPLIED GENETICS, vol. 101, no. 5-6, October 2000 (2000-10), pages 823-829, XP002227768 ISSN: 0040-5752**
• **JIN Q S ET AL: "Identification and potential use of a RAPD marker for aroma in rice." JOURNAL OF GENETICS & BREEDING, vol. 50, no. 4, 1996, pages 367-370, XP009003991 ISSN: 0394-9257**
• **TAN Y F ET AL: "Mapping quantitative trait loci for milling quality, protein content and color characteristics of rice using a recombinant inbred line population derived from an elite rice hybrid." THEORETICAL AND APPLIED GENETICS, vol. 103, no. 6-7, November 2001 (2001-11), pages 1037-1045, XP002227769 November, 2001 ISSN: 0040-5752**

EP 1 298 222 B1

- GARLAND S ET AL: "PCR-based molecular markers for the fragrance gene in rice (Oryza sativa. L.)." THEORETICAL AND APPLIED GENETICS, vol. 101, no. 3, August 2000 (2000-08), pages 364-371, XP002227773 ISSN: 0040-5752
- GOTO MASAHIRO ET AL: "Comparison of palatability and physicochemical properties of boiled rice among red rice, Koshihikari and Minenishiki." JOURNAL OF THE JAPANESE SOCIETY FOR FOOD SCIENCE AND TECHNOLOGY, vol. 43, no. 7, 1996, pages 821-824, XP009004113 ISSN: 0029-0394
- HE P ET AL: "Genetic analysis of rice grain quality." THEORETICAL AND APPLIED GENETICS, vol. 98, no. 3-4, March 1999 (1999-03), pages 502-508, XP002227770 ISSN: 0040-5752
- BRES-PATRY C ET AL: "Heredity and genetic mapping of domestication-related traits in a temperate japonica weedy rice." THEORETICAL AND APPLIED GENETICS, vol. 102, no. 1, January 2001 (2001-01), pages 118-126, XP002227771 ISSN: 0040-5752
- ALI M L ET AL: "Mapping QTLs for root traits in a recombinant inbred population from two indica ecotypes in rice." THEORETICAL AND APPLIED GENETICS, vol. 101, no. 5-6, October 2000 (2000-10), pages 756-766, XP002227772 ISSN: 0040-5752
- AHN S N ET AL: "RFLP analysis of genomic regions associated with cooked-kernel elongation in rice." THEORETICAL AND APPLIED GENETICS, vol. 87, no. 1-2, 1993, pages 27-32, XP009004035 ISSN: 0040-5752
- ZHUANG J -Y ET AL: "Analysis of QTL X times environment interaction for yield components and plant height in rice." THEORETICAL AND APPLIED GENETICS, vol. 95, no. 5-6, October 1997 (1997-10), pages 799-808, XP002227774 ISSN: 0040-5752
- XIAO J., LI J., YUAN L., TANKSLEY S.D.: "Identification of QTLs affecting traits og agronomic importance in a recombinant inbred population derived from a subspecific rice cross." THEORETICAL AND APPLIED GENETICS, vol. 92, 1996, pages 230-244, XP009004066
- XIAO J., LI J., YUAN L. AND MCCOUCH S.R.: "Genetic diversity and its relationship to hybrid performance and heterosis in rice as revealed by PCR-based markers." THEORETICAL AND APPLIED GENETICS, vol. 92, 1996, pages 637-643, XP009004067
- MCCOUCH S.R., KOCHERT G., YU Z.H., WANG Z.Y., KHUSH G.S., COFFMAN W.R. AND TANKSLEY S.D.: "Molecular mapping of rice chromosomes." THEORETICAL AND APPLIED GENETICS, vol. 76, 1988, pages 815-826, XP002093980
- SINGH RK, SINGH US, KHUSH GS AND ROHILLA RASHMI: "Aromatic rices. Chap. 5: Genetics and biotechnology of quality traits in aromatic rices." 2000 , SCIENCE PUBLISHERS INC. , ENFIELD, NEW HAMPSHIRE, 03748, USA XP001122119 * page 47 - page 69 *
- GRAHAM R: "A proposal for IRRI to establish a grain quality and nutrition resarch center." 2002 , INTERNATIONAL RICE RESEARCH INSTITUTE , LOS BAÑOS, PHILIPPINES XP002227777
- INOUE T ET AL: "Sequence-tagged sites (STSs) as standard landmarkers in the rice genome." THEORETICAL AND APPLIED GENETICS, vol. 89, no. 6, 1994, pages 728-734, XP009004275 ISSN: 0040-5752

SEQUENCE LISTING

<110> National Food Research Institute
<120> DNA-level rice palatability evaluation method, and method of selecting palatable rice through analysis of half grain of unhulled/unpolished rice
<130> P131218K
<160> 92

<210> 1
<211> 30
<212> DNA
<213> Artificial Sequence

<400> 1
ccagctgaac gcctgtacta caagaattaa          30

<210> 2
<211> 30
<212> DNA
<213> Artificial Sequence

<400> 2
ccagctgtac gtcttcccca gcgccggcgg          30

<210> 3
<211> 21
<212> DNA
<213> Artificial Sequence

<400> 3
ccagctgtac gcctgtacta c          21

<210> 4
<211> 22
<212> DNA
<213> Artificial Sequence

<400> 4
ccagctgtac gtcttcccca gc          22

<210> 5
<211> 30
<212> DNA
<213> Artificial Sequence

<400> 5
tgcctcgcac cagaaatagt ataatcccaa          30

<210> 6
<211> 30
<212> DNA
<213> Artificial Sequence

<400> 6
tgcctcgcac catgaggtgt ggccgagtac          30

<210> 7
<211> 19
<212> DNA
<213> Artificial Sequence

<400> 7
tgcctcgcac cagaaatag          19


<210> 8
<211> 16
<212> DNA
<213> Artificial Sequence


<400> 8
tgcctcgcac catgag          16


<210> 9
<211> 30
<212> DNA
<213> Artificial Sequence


<400> 9
gtttcgctcc tacagtaatt aaggggctat          30


<210> 10
<211> 30
<212> DNA
<213> Artificial Sequence


<400> 10
gtttcgctcc catgcaatct gcaaaagttt          30


<210> 11
<211> 25
<212> DNA
<213> Artificial Sequence


<400> 11
gtttcgctcc tacagtaatt aaggg          25


<210> 12
<211> 20
<212> DNA
<213> Artificial Sequence


<400> 12
gtttcgctcc catgcaatct          20


<210> 13
<211> 30
<212> DNA
<213> Artificial Sequence


<400> 13
caggtgtggg ttacaaggat gacccttggg          30


<210> 14
<211> 30
<212> DNA
<213> Artificial Sequence


<400> 14
caggtgtggg ttcacggcct tgattaataa          30

<210> 15
<211> 22
<212> DNA
<213> Artificial Sequence

<400> 15
caggtgtggg ttacaaggat ga          22


<210> 16
<211> 17
<212> DNA
<213> Artificial Sequence

<400> 16
caggtgtggg ttcacgg          17


<210> 17
<211> 30
<212> DNA
<213> Artificial Sequence

<400> 17
ccacagcagt gcttcatgtc atgtagaata          30


<210> 18
<211> 30
<212> DNA
<213> Artificial Sequence

<400> 18
ccacagcagt tcaaatacac caggaatttc          30


<210> 19
<211> 15
<212> DNA
<213> Artificial Sequence

<400> 19
ccacagcagt gcttc          15


<210> 20
<211> 21
<212> DNA
<213> Artificial Sequence

<400> 20
ccacagcagt gcttcatgtc a          21


<210> 21
<211> 30
<212> DNA
<213> Artificial Sequence

<400> 21
tggccggcat gactcacata cccaacatat          30


<210> 22
<211> 30

<212> DNA
<213> Artificial Sequence

<400> 22
actggccggc atcaagacca accaatttgg          30

<210> 23
<211> 17
<212> DNA
<213> Artificial Sequence

<400> 23
tggccggcat gactcac          17

<210> 24
<211> 18
<212> DNA
<213> Artificial Sequence

<400> 24
actggccggc atcaagac          18

<210> 25
<211> 30
<212> DNA
<213> Artificial Sequence

<400> 25
ggaatggaac cgaagtggag ctattccctg          30

<210> 26
<211> 30
<212> DNA
<213> Artificial Sequence

<400> 26
ggaatggaac cgccgtaaac ttgaatgcta          30

<210> 27
<211> 20
<212> DNA
<213> Artificial Sequence

<400> 27
ggaatggaac cgaagtggag          20

<210> 28
<211> 21
<212> DNA
<213> Artificial Sequence

<400> 28
ggaatggaac cgccgtaaac t          21

<210> 29
<211> 30
<212> DNA
<213> Artificial Sequence

<400> 29
tacctggttg atgtatacag atctggttat          30

<210> 30
<211> 30
<212> DNA
<213> Artificial Sequence

<400> 30
atccctcgat ccctctagca ttatatcctc          30

<210> 31
<211> 28
<212> DNA
<213> Artificial Sequence

<400> 31
tacctggttg atgtatacag atctggtt          28

<210> 32
<211> 24
<212> DNA
<213> Artificial Sequence

<400> 32
atccctcgat ccctctagca ttat          24

<210> 33
<211> 30
<212> DNA
<213> Artificial Sequence

<400> 33
accactccat atatatcatc caaagttcta          30

<210> 34
<211> 30
<212> DNA
<213> Artificial Sequence

<400> 34
accactccat atcaccacaa ggcgtttagg          30

<210> 35
<211> 25
<212> DNA
<213> Artificial Sequence

<400> 35
accactccat atatatcatc caaag          25

<210> 36
<211> 22
<212> DNA
<213> Artificial Sequence

<400> 36
accactccat atcaccacaa gg          22

<210> 37
<211> 30
<212> DNA
<213> Artificial Sequence

<400> 37
gagaccgata tgcgattcgc ggcattggac        30

<210> 38
<211> 30
<212> DNA
<213> Artificial Sequence

<400> 38
gtggtgttta gatccagaga cttaacttta        30

<210> 39
<211> 18
<212> DNA
<213> Artificial Sequence

<400> 39
gagaccgata tgcgattc        18

<210> 40
<211> 24
<212> DNA
<213> Artificial Sequence

<400> 40
gtggtgttta gatccagaga ctta        24

<210> 41
<211> 30
<212> DNA
<213> Artificial Sequence

<400> 41
ctcactcaaa tttacagtgc attttcttgt        30

<210> 42
<211> 30
<212> DNA
<213> Artificial Sequence

<400> 42
agggccatga tacaagactc tgttctgtag        30

<210> 43
<211> 27
<212> DNA
<213> Artificial Sequence

<400> 43
ctcactcaaa tttacagtgc attttct        27

<210> 44
<211> 23

EP 1 298 222 B1

<212> DNA
<213> Artificial Sequence

<400> 44
agggccatga tacaagactc tgt          23

<210> 45
<211> 30
<212> DNA
<213> Artificial Sequence

<400> 45
ggccgtcgtt ctgcgatggt ctccaagaat          30

<210> 46
<211> 30
<212> DNA
<213> Artificial Sequence

<400> 46
ggagaatccc acagtaagtt tttctttgtt          30

<210> 47
<211> 17
<212> DNA
<213> Artificial Sequence

<400> 47
ggcggtcgtt ctgcgat          17

<210> 48
<211> 28
<212> DNA
<213> Artificial Sequence

<400> 48
ggagaatccc acagtaagtt tttctttg          28

<210> 49
<211> 30
<212> DNA
<213> Artificial Sequence

<400> 49
gtcggagtgg tcagaccggg ctagcttttg          30

<210> 50
<211> 30
<212> DNA
<213> Artificial Sequence

<400> 50
gtcggagtgg atggagtagc ggtgggtgtg          30

<210> 51
<211> 18
<212> DNA
<213> Artificial Sequence

<400> 51
gtcggagtgg tcagaccg          18

<210> 52
<211> 20
<212> DNA
<213> Artificial Sequence

<400> 52
gtcggagtgg atggagtagc          20

<210> 53
<211> 30
<212> DNA
<213> Artificial Sequence

<400> 53
acaacgcctc cgatgatcga accatatctt          30

<210> 54
<211> 30
<212> DNA
<213> Artificial Sequence

<400> 54
acaacgcctc cgacaacaag attttctcct          30

<210> 55
<211> 16
<212> DNA
<213> Artificial Sequence

<400> 55
acaacgcctc cgatga          16

<210> 56
<211> 15
<212> DNA
<213> Artificial Sequence

<400> 56
acaacgcctc cgaca          15

<210> 57
<211> 30
<212> DNA
<213> Artificial Sequence

<400> 57
gtccactgtg accacaacat ttcttccagc          30

<210> 58
<211> 30
<212> DNA
<213> Artificial Sequence

<400> 58
gtccactgtg gggattgttc cataaaagat          30

<210> 59
<211> 20
<212> DNA
<213> Artificial Sequence

<400> 59
gtccactgtg accacaacat        20

<210> 60
<211> 20
<212> DNA
<213> Artificial Sequence

<400> 60
gtccactgtg gggattgttt        20

<210> 61
<211> 30
<212> DNA
<213> Artificial Sequence

<400> 61
aacgggccaa aaacggaggt cgtatggagc        30

<210> 62
<211> 30
<212> DNA
<213> Artificial Sequence

<400> 62
aacgggccaa cgcagccatt aaagagaaat        30

<210> 63
<211> 20
<212> DNA
<213> Artificial Sequence

<400> 63
aacgggccaa aaacggaggt        20

<210> 64
<211> 15
<212> DNA
<213> Artificial Sequence

<400> 64
aacgggccaa cgcag        15

<210> 65
<211> 30
<212> DNA
<213> Artificial Sequence

<400> 65
acaacggtcc gtccttgctt aggaaaaggc        30

<210> 66
<211> 30

<212> DNA
<213> Artificial Sequence

<400> 66
acaacggtcc aacagatact tttgaaaaac          30

<210> 67
<211> 20
<212> DNA
<213> Artificial Sequence

<400> 67
acaacggtcc gtccttgctt          20

<210> 68
<211> 25
<212> DNA
<213> Artificial Sequence

<400> 68
acaacggtcc aacagatact tttga          25

<210> 69
<211> 30
<212> DNA
<213> Artificial Sequence

<400> 69
agtgcagcca ttatatagga ctaacaagga          30

<210> 70
<211> 30
<212> DNA
<213> Artificial Sequence

<400> 70
agtgcagcca aaccagaaga aagccatgtt          30

<210> 71
<211> 25
<212> DNA
<213> Artificial Sequence

<400> 71
agtgcagcca ttatatagga ctaac          25

<210> 72
<211> 20
<212> DNA
<213> Artificial Sequence

<400> 72
agtgcagcca aaccagaaga          20

<210> 73
<211> 30
<212> DNA
<213> Artificial Sequence

<400> 73
gtcgttcctg tggttaggac agggtcgcaa       30

<210> 74
<211> 30
<212> DNA
<213> Artificial Sequence

<400> 74
gtcgttcctg ctggtgtctc agatcgttcg       30

<210> 75
<211> 25
<212> DNA
<213> Artificial Sequence

<400> 75
gtcgttcctg tggttaggac agggt       25

<210> 76
<211> 24
<212> DNA
<213> Artificial Sequence

<400> 76
gtcgttcctg ctggtgtctc agat       24

<210> 77
<211> 30
<212> DNA
<213> Artificial Sequence

<400> 77
aacggcgaca taaaataagt tgttacatgt       30

<210> 78
<211> 30
<212> DNA
<213> Artificial Sequence

<400> 78
aacggcgaca gtggcatgct cgatgacgac       30

<210> 79
<211> 22
<212> DNA
<213> Artificial Sequence

<400> 79
aacggcgaca taaaataagt tg       22

<210> 80
<211> 25
<212> DNA
<213> Artificial Sequence

<400> 80
aacggcgaca gtggcatgct cgatg       25

<210> 81
<211> 30
<212> DNA
<213> Artificial Sequence

<400> 81
ggtgaacgct gtagttggaa tataagtata          30

<210> 82
<211> 30
<212> DNA
<213> Artificial Sequence

<400> 82
ggtgaacgct cagatttaaa tataattagt          30

<210> 83
<211> 24
<212> DNA
<213> Artificial Sequence

<400> 83
ggtgaacgct gtagttggaa tata          24

<210> 84
<211> 26
<212> DNA
<213> Artificial Sequence

<400> 84
ggtgaacgct cagatttaaa tataat          26

<210> 85
<211> 30
<212> DNA
<213> Artificial Sequence

<400> 85
ccgcagttag atgcaccatt agaattgctt          30

<210> 86
<211> 30
<212> DNA
<213> Artificial Sequence

<400> 86
ccgcagttag atcaagtggc aaggttccat          30

<210> 87
<211> 20
<212> DNA
<213> Artificial Sequence

<400> 87
ccgcagttag atgcaccatt          20

<210> 88
<211> 20

<212> DNA
<213> Artificial Sequence

<400> 88
ccgcagttag atcaagtggc          20

<210> 89
<211> 40
<212> DNA
<213> Artificial Sequence

<400> 89
gtcgttcctg tggttaggac agggtcgcaa attcagtctt          40

<210> 90
<211> 40
<212> DNA
<213> Artificial Sequence

<400> 90
gtcgttcctg ctggtgtctc agatcgttcg ggggcttgcg          40

<210> 91
<211> 12
<212> DNA
<213> Artificial Sequence

<400> 91
gtcgttcctg tg          12

<210> 92
<211> 12
<212> DNA
<213> Artificial Sequence

<400> 92
gtcgttcctg ct          12

**Description**

FIELD OF THE INVENTION

The present invention relates to a DNA-level technique of evaluating the palatability of rice and selecting palatable rice. Precisely, the invention relates to a DNA-level rice palatability evaluation method for selecting palatable rice, which comprises amplifying the DNA extracted from a rice plant, unhulled rice, unpolished rice, polished rice, boiled rice, rice cake or ground powder thereof through PCR in the presence of at least two STS primer pairs, selecting a DNA band of close correlation with palatability evaluation from the resulting amplified DNA bands, and using it as a DNA marker for palatability evaluation to select palatable rice. Palatable rice referred herein means rice with good taste and high quality.

BACKGROUND OF THE INVENTION

Rice, wheat and corn are said to be the three most important cereals in many countries including Japan, and are considered very important for the principal food for more than a half of persons in the world. Recently, not only the quantity of rice for food but also the quality thereof , or that is, the palatability of boiled rice for meals has been considered important. In the global rice situation, soft textured and aromatic rice such as "Basmati 370" in India and Pakistan, "Jasmine rice" in Thailand and "Koshihikari" in Japan is much desired and is traded at high price.

For rice palatability evaluation, there are two main methods. One is an organoleptic test (sensory test) of trying boiled rice to evaluate its eating quality; and the other is a physicochemical test of analyzing the chemical components of rice or boiled rice and measuring the physical properties thereof to estimate the palatability of the rice from the data. The two have both merits and demerits. At present, therefore, one or both of the two methods are employed for rice palatability evaluation.

However, the organoleptic test and the physicochemical test both require at least 50 to 500 g of rice to be tested therein. According to the test methods, therefore, it is impossible to estimate the palatability of rice of a certain lot or a certain variety or line from a small quantity of rice, especially from only one grain of rice.

We, the present inventors have heretofore made various studies relating to rice palatability evaluation, and have announced them in publications, for example, in *Food Industry,* Vol. 42, No. 17, pp. 55-61. However, such our prior techniques are also for organoleptic test or physicochemical test that requires a predetermined quantity of rice to be tested, and according to these, it is still impossible to estimate the palatability of rice by non-disruptively testing a small quantity of rice, especially only one grain of rice.

Heretofore, we have also studied various DNA-level techniques of rice variety discrimination through RAPD method or through a process of using STS primers, and have announced them in publications, for example, in the *Journal of the Food Science and Technology in Japan,* Vol. 46, No. 3, pp. 117-122, and filed patent applications for them (e.g. , Japanese Patent Laid-Open No. 2001-95589). However, these our prior techniques are directed to registered varieties of rice and are for discriminating the rice varieties, combined with DNA markers for variety discrimination irrespective of palatability. Anyhow, the techniques are not for DNA-level palatability evaluation directed to rice in a broad range including unidentified varieties of rice produced in various districts in the world, new varieties of rice now being bred, and other varieties of rice which are now on the market but of which the palatability is not as yet evaluated.

Using rice seedlings and a large quantity of rice as samples, studies of RFLP (restriction fragment length polymorphism)-based genetic markers have heretofore been made for searching for rice genes that are resistant to phytopathogens and harmful insects. In the RFLP-based process, however, DNA fragments prepared by processing sample DNAs with various restriction endonucleases are analyzed for DNA polymorphism, and the process is not applicable to non-disruptive rice palatability evaluation and palatable rice selection as in the present invention in which a small quantity of rice, especially one grain of rice is sampled.

Therefore, according to the related techniques in the art, it has heretofore been impossible to evaluate the palatability of rice by sampling a small quantity of rice, especially one grain of rice and analyzing it with a DNA marker.

SUMMARY OF THE INVENTION

The present invention is to provide a DNA-level rice palatability evaluation method of using a very small quantity of rice, especially a half or one grain of rice as a sample. This is to evaluate the palatability of rice, not disrupting the embryo of a rice grain that is to be the origin of a rice plant of the coming generation, and to select palatable rice.

We, the present inventors have assiduously studied to attain the object as above, and, as a result, have found that palatable rice can be selected from various rice samples by extracting DNAs from each rice sample, amplifying them through PCR in the presence of suitable primers, selecting DNA bands of close correlation with palatability evaluation from the resulting amplified DNA bands, and using them as DNA markers for palatability evaluation to select palatable rice. On the basis of this finding, we have completed the present invention.

Specifically, the invention provides a DNA-level rice palatability evaluation method for selecting palatable rice, which comprises amplifying the DNA extracted from a rice plant, unhulled rice, unpolished rice, polished rice, boiled rice, rice cake or ground powder thereof through PCR in the presence of at least two STS primer pairs, selecting DNA bands of close correlation with palatability evaluation from the resulting amplified DNA bands, and using it as DNA markers for palatability evaluation to select palatable rice.

In one embodiment of the DNA-level rice palatability evaluation method of the invention, the rice sample from which its DNA is extracted is an embryo-free half grain of unhulled or unpolished rice, and after the palatable rice has been selected by the use of the DNA markers obtained through PCR, the other half thereof having an embryo is allowed to germinate and grow into a rice plant to thereby selectively breed the thus-selected variety of palatable rice.

In another embodiment of the DNA-level rice palatability evaluation method of the invention, the PCR is effected through at least two STS primer pairs, and the DNA bands of close positive and/or negative correlation with palatability evaluation in organoleptic examination and/or physicochemical examination is used as the DNA markers for palatability evaluation to select palatable rice.

In still another embodiment of the DNA-level rice palatability evaluation method of the invention, the DNA marker of close positive and/or negative correlation with palatability evaluation in organoleptic examination and/or physicochemical examination is selectively used to select palatable rice on the basis of the presence or absence of the marker in the sample.

In still another embodiment of the DNA-level rice palatability evaluation method of the invention, the rice palatability evaluation in physicochemical examination is effected by measuring the properties of boiled rice.

In still another embodiment of the DNA-level rice palatability evaluation method of the invention, one or more STS primers selected from a primer group of A6, A7, B1, B7, B18, B43, E22, E30, F6, G4, G22, G28, J6, M2CG, M11, P3, P5, Q16, S13, T8, T16 and WK9 are used in PCR.

## BRIEF DESCRIPTION OF THE DRAWINGS

Fig. 1 shows DNA band patterns seen in electrophoresis. In this, M indicates a marker; lane 1 is "Koshihikari"; lane 2 is "Hitomebore"; lane 3 is "Hinohikari"; lane 4 is "Akitakomachi"; lane 5 is "Kirara 397"; lane 6 is "Kinuhikari"; lane 7 is "Hoshinoyume"; lane 8 is "Haenuki"; lane 9 is "Mutsuhomare"; lane 10 is "Nipponbare" ; lane 11 is "Sasanishiki" ; lane 12 is "Tsugaruroman"; lane 13 is "Hanaechizen"; lane 14 is "Yumetsukushi"; lane 15 is "Hatsushimo"; lane 16 is "Asanohikari"; lane 17 is "Tsukinohikari"; lane 18 is "Aichinokaori"; lane 19 is "Matsuribare"; lane 20 is "Akiho".

Fig. 2 is a graph showing a correlation between the found value and the predicted value of the palatability of rice harvested in 1997.

Fig. 3 is a graph showing a correlation between the found value and the predicted value of the palatability of rice harvested in 1998.

Fig. 4 is a graph showing a correlation between the found value and the predicted value of the palatability of rice harvested in 1999.

Fig. 5 is a graph showing a correlation between the found value and the predicted value of the palatability of rice in Example 2.

Fig. 6 is a graph showing the data of the principal components of rice analyzed in Example 2.

Fig. 7 is a graph showing a correlation between the found value and the predicted value of the palatability of rice in Example 3.

Fig. 8 is a graph showing a correlation between the found value and the predicted value of the physical property of rice in Example 4.

Fig. 9 is a graph showing a correlation between the found value and the predicted value of the physical property of rice in Example 5.

Fig. 10 is a graph showing a correlation between the found value and the predicted value of the physical property of rice in Example 6.

## DETAILED DESCRIPTION OF THE INVENTION

The invention is described in detail hereinunder.

In the invention, the palatability of rice is meant to indicate the palatability of boiled rice prepared by boiling polished rice for meals. It is known that the palatability of boiled rice varies in different countries and different districts and changes with the times, depending on the environment at meal time and on the condition of panelists whether they are hungry or not.

However, when many panelists try boiled rice prepared under a predetermined condition, they evaluate it according to their likes and dislikes. For example, most Japanese like "soft, glutinous and bright boiled rice"; but on the contrary, most Indians like "relatively stiff and non-glutinous boiled rice hard to chew".

The palatability of rice referred to herein includes the difference in liking for boiled rice between individuals. For it, for example, referred to are the rice palatability ranking by the Japan Association of Grain Inspection, which is annually carried out on the basis of the organoleptic examination method developed by the former National Food Research Institute, Ministry of Agriculture, Forestry and Fisheries and of which the results are announced; the results of palatability tests that are carried out in rice-breeding laboratories of agricultural experiment stations in various districts; and the results of physicochemical examination for the palatability of rice that is described hereinunder.

The organoleptic examination of the palatability of rice for the invention is as follows: From 4 to 50 (generally 24) panelists try samples of boiled rice. Comparing them with a reference sample of standard boiled rice, the panelists evaluate the test samples in point of the matters of "total evaluation", "hardness", "stickiness", "appearance", "palatability" and "aroma" as to whether the test samples are "not good", "average" or "good". All the panelists' data are statistically processed through significance level differentiation from average data or through two-way layout and dispersion analysis to thereby evaluate the palatability of the rice samples tested. For example, the above-mentioned method by the Japan Association of Grain Inspection and the palatability examination methods that are carried out in national and public rice-breeding laboratories all over Japan correspond to the organoleptic examination.

In the invention, physicochemical examination of rice is for estimating the palatability of rice from its data, and is as follows: The chemical components of rice are analyzed, for example, by measuring the protein content or the amylose content thereof. A rice grain or rice powder is exposed to visible light to obtain its near-IR spectrum. Rice powder is tested for its gelatinization. While boiled rice is checked for its behavior. The properties of boiled rice are measured. The explanatory variables of the individual test results and data are processed through multivariate analysis such as multiple regression analysis or neural network analysis. This gives an estimated palatability value of the rice sample tested, and this is the physicochemical examination of rice for estimating the palatability of rice from its data in the invention.

The measurement of the physical properties of boiled rice in the invention is as follows: The hardness and the stickiness of boiled rice that are said to have close relation to the palatability thereof are measured with a tensile compression tester such as Texturometer, Rheometer, Rheograph or Tensipresser or with a Viscoelastometer, such as Rheolographmicro. Various samples of boiled rice, for example, about 1 to 3 grains or more thereof and even rice balls may be tried for measuring their physical properties.

For extracting the DNA from a rice plant, unhulled rice, unpolished rice, polished rice, boiled rice, rice cake or ground powder thereof in the invention, employable are any ordinary methods, for example, a method using cetyltrimethylammonium bromide (hereinafter referred to as CTAB method), an alkali SDS method, a phenol method, an enzymatic method or a benzyl chloride method. For the DNA extraction, also employable are commercially-available DNA extraction kits. In the invention, preferred is the CTAB method or enzymatic method as in the Examples mentioned hereinunder.

Concretely, for example, a CTAB solution (0.1 M tris-HCl, 2 mM disodium ethylenediaminetetraacetate (EDTA), 1.4 M NaCl (pH 8.0)) is added to a sample and stirred. This is put into an incubator, and the CTAB solution is again added thereto and left as it is for a predetermined period of time to extract the genome DNA from the sample.

In case where the sample is from boiled rice or rice cake, its genome DNA may be extracted according to an enzyme extraction process as in Japanese Patent No. 3,048,149. Concretely, the sample is powdered, the powder is dissolved in buffer solution and processed with heat-resistant amylase, and a genome gene is extracted from it.

In case where the sample is from unhulled rice or unpolished rice, its genome DNA may be extracted as follows: Each unhulled or unpolished grain of rice to be tested is halved with a cutter or a knife, and its genome DNA is extracted from an embryo (germ) -free half of the grain and tried for palatability evaluation. As will be described hereinunder, the other half thereof having an embryo (germ) is allowed to germinate and grow into a rice plant. In that manner, plants of palatable rice can be selectively bred.

If desired, the thus-extracted DNA may be purified, for example, through treatment with chloroform/isoamyl alcohol, or isopropanol precipitation, or protein removal with phenol/chloroform, or ethanol precipitation. For the purification, preferred is treatment with chloroform/isoamyl alcohol. Concretely, chloroform/isoamyl alcohol (24/1) is added to a DNA extract, stirred and centrifuged; a DNA precipitant (1% CTAB solution, 20 mM tris-HCl, 10 mM EDTA, pH 8.0) is added to the resulting supernatant to precipitate the DNA; this is again centrifuged, and the resulting DNA precipitate is extracted with 1 M NaCl ; and the DNA extract is washed with isopropyl alcohol and ethanol, then precipitated, and dissolved in a TEbuffer. The process gives a purified DNA sample solution.

Subsequently, the genome DNA obtained as in the above is subjected to PCR in which it serves as a template in the presence of a random primer. This is for amplifying the base sequence of the genome DNA, which is for variety discrimination and is the basis in planning the STS primers for the next PCR.

PCR in the invention is chain reaction with DNA polymerase for DNA replication. One cycle of PCR comprises three steps of denaturation, annealing and chain-extension. The denaturation step comprises heating the template DNA at a high temperature falling between 90 and 96°C in the presence of various gene fragments (primers) to thereby separate the double-stranded template DNA into the individual single strands; the annealing step comprises bonding the primer to the DNA at 30 to 75°C; and the chain-extension step comprises extending the DNA molecule from its part to which the primer bonds, at 70 to 75°C in the presence of a heat-resistant DNA polymerase. In the invention, from 20 to 50 cycles of such PCR are repeated to amplify the template DNA to about 1,000,000 to 10,000,000,000 times.

The STS (sequence-tagged site) primer for use in the invention is described. This is a pair of primers having the function of amplifying the base sequence for variety discrimination in DNA. As so mentioned hereinabove, the STS primer is designed on the basis of the base sequence of DNA that gives a variety discrimination band. The base sequence is obtained through PCR of the DNA of a rice sample according to RAPD method. Concretely, a genome DNA of an object rice sample is subj ected to PCR in which it serves as a template in the presence of a random primer, and a part of the DNA that has given a variety discrimination band is sequenced. Of the base sequence, from 8 to 50 base residues on the forward side of the random primer and on the reverse side thereof are selected for a pair of primers. The pair primers are used in PCR for variety discrimination along with the template DNA of the object grain.

In that manner, the STS primer is specifically selected from the random primer that has plural sites to receive the template DNA. Therefore, in palatability evaluation PCR of the template DNA, the thus-selected STS primer selectively bonds to only the base sequence of the template DNA to give a variety discrimination band.

The DNA thus amplified through such PCR is subjected to electrophoresis. This is for detecting the band of the base sequence for palatability evaluation from the amplified products, and the thus-detected base sequence is to be the basis in designing the intended STS primer.

Based on the thus-detected band that indicates the base sequence for palatability evaluation, a pair of primers are constructed as follows:

The DNA fragment having given the band of palatability evaluation is cut out of the gel to extract and collect the DNA, and this is transformed into cells of E. coli. With the DNA therein, the transformant cells are grown. Next, the plasmid DNA is extracted out of the cells according to an alkaline miniprep process. Serving as a template DNA, the plasmid DNA is amplified through PCR, and then this is sequenced by the use of an automatic DNA sequencer.

Based on the thus-sequenced DNA, STS primers are designed. In the previous PCR with a random primer, the sequence that includes the site of the rice sample-derived DNA, or the template DNA to which the random primer has bonded should be the same as or complimentary (homologous) to that of the random primer. In other words, the DNA base sequence of high palatability evaluation that has been cut and extracted out of the template DNA (this is to be the basis of the STS primer in PCR) should have a sequence part that is the same as or homologous to the base sequence of the random primer at its both ends.

Accordingly, from both the forward side and the reverse side of the DNA base sequence of high palatability evaluation, STS primers having a suitable sequence and a suitable length that are useful for palatable rice determination can be designed.

As so mentioned hereinabove, the STS primer for use in the invention is a pair of forward-reverse primers (pair primer), and this is designed by sequencing the DNA fragment extracted on the basis of the discrimination band in PCR followed by constructing its base sequence so that it sandwiches the discrimination band-giving DNA sequence between its 5' -side and 3'-side and therefore it can amplify only the thus-sandwiched, discrimination band-giving DNA sequence in PCR. Preferably, the pair STS primers for use in the invention have the same or homologous sequence.

Regarding its size (number of component base), it is desirable that the STS primer for use in the invention has from 8 to 50 bases, more preferably from 15 to 30 bases. If its size oversteps the range, it is unfavorable since the primer could not well bond to the template DNA, and after bonded thereto, the primer could not well dissociate from it, and, in addition, the DNA does not give a discrimination band in PCR and will be therefore useless for palatability evaluation. On the other hand, if its size is smaller than the range, it is also unfavorable since the primer may non-specifically bond to some other unintended DNA fragments and may be mismatched with them, and, as a result, will be useless for palatability evaluation because the band expression frequency not indicating the intended discrimination bands will increase. After all, such small-sized primers will be useless for rapid and simple palatability evaluation in PCR using plural primers.

Through PCR experiments made to the effect as above, we, the present inventors have obtained various palatability evaluation bands effective for discriminating rice varieties from each other. Table 1 below shows a correlation between some rice palatability evaluation bands and rice varieties that may be discriminated from each other on the basis of the bands.

In the invention, the DNA band for palatability evaluation is meant to indicate the band of a DNA which, after amplified through PCR, gives in electrophoresis a pattern enough to differentiate the palatability of the sample rice from any other different varieties.

Table 1-1

| Rice variety Discrimination Band | A6 | A7 | B1 | B7 | B18 | B43 |
|---|---|---|---|---|---|---|
| Band length (kbp) | 0.7 | 0.7 | 0.5 | 0.5 | 1.0 | 0.9 |
| Koshihikari | - | + | - | - | + | + |
| Hitomebore | - | + | - | - | + | + |
| Hinohikari | - | + | + | - | + | - |
| Akitakomachi | - | + | - | - | + | + |
| Kirara 397 | - | + | - | - | - | - |
| Kinuhikari | + | + | + | + | - | - |
| Hoshinoyume | - | + | - | - | - | - |
| Haenuki | + | + | - | - | - | - |
| Mutsuhomare | - | + | + | + | - | + |
| Nipponbare | - | - | + | - | - | + |
| Sasanishiki | - | + | - | + | - | + |
| Tsugaruroman | - | + | - | + | - | - |
| Hanaechizen | - | - | - | - | - | - |
| Yumetsukushi | - | + | + | - | + | + |
| Hatsushimo | + | + | + | - | - | + |
| Asanohikari | - | - | + | - | - | - |
| Tsukinohikari | - | - | + | - | - | + |
| Aichinokaori | + | + | - | - | - | + |
| Matsuribare | - | - | + | - | - | - |
| Akiho | - | + | - | - | - | + |

+: Discrimination band appeared in electrophoresis after PCR.
-: Discrimination band did not appear in electrophoresis after PCR.

Table 1-2

| Rice Variety Discrimination Band | E22 | E30 | F6 | G4 | G22 | G28 |
|---|---|---|---|---|---|---|
| band length (kbp) | 1.9 | 0.8 | 1.2 | 0.9 | 0.7 | 0.4 |
| Koshihikari | + | - | - | - | + | + |
| Hitomebore | + | + | - | - | + | + |
| Hinohikari | - | - | - | - | + | - |
| Akitakomachi | + | - | - | - | - | + |
| Kirara 397 | + | - | - | + | + | + |
| Kinuhikari | + | - | - | - | - | + |
| Hoshinoyume | - | - | - | - | + | + |
| Haenuki | + | - | - | - | - | + |
| Mutsuhomare | + | + | + | - | - | + |
| Nipponbare | - | - | + | - | - | - |
| Sasanishiki | - | - | - | - | + | - |

(continued)

| Rice Variety Discrimination Band | E22 | E30 | F6 | G4 | G22 | G28 |
|---|---|---|---|---|---|---|
| band length (kbp) | 1.9 | 0.8 | 1.2 | 0.9 | 0.7 | 0.4 |
| Tsugaruroman | - | - | - | - | - | + |
| Hanaechizen | - | - | - | - | - | + |
| Yumetsukushi | - | - | - | - | - | + |
| Hatsushimo | + | - | - | - | - | - |
| Asanohikari | - | - | - | - | - | - |
| Tsukinohikari | - | - | + | - | - | - |
| Aichinokaori | + | - | + | - | - | - |
| Matsuribare | - | - | + | - | + | - |
| Akiho | - | - | - | + | + | - |
| +: Discrimination band appeared in electrophoresis after PCR. -: Discrimination band did not appear in electrophoresis after PCR. | | | | | | |

Table 1-3

| Rice Variety Discrimination Band | J6 | M2CG | M11 | P3 | P5 | Q16 |
|---|---|---|---|---|---|---|
| band length (kbp) | 0.9 | 1.2 | 0.7 | 0.5 | 0.4 | 0.6 |
| Koshihikari | + | - | + | + | + | + |
| Hitomebore | + | + | + | - | + | + |
| Hinohikari | + | - | + | + | + | + |
| Akitakomachi | + | - | + | - | + | + |
| Kirara 397 | + | + | - | + | - | + |
| Kinuhikari | + | - | + | + | + | + |
| Hoshinoyume | + | + | - | + | - | - |
| Haenuki | + | + | + | - | + | + |
| Mutsuhomare | + | + | - | - | - | + |
| Nipponbare | + | + | - | + | - | - |
| Sasanishiki | - | - | + | - | - | + |
| Tsugaruroman | + | - | + | - | - | - |
| Hanaechizen | + | + | + | + | + | - |
| Yumetsukushi | + | - | - | + | + | - |
| Hatsushimo | + | + | + | + | + | - |
| Asanohikari | + | - | + | + | - | - |
| Tsukinohikari | + | + | - | + | - | - |
| Aichinokaori | + | + | + | + | - | - |
| Matsuribare | + | - | + | + | - | - |

(continued)

| Rice Variety Discrimination Band | J6 | M2CG | M11 | P3 | P5 | Q16 |
|---|---|---|---|---|---|---|
| band length (kbp) | 0.9 | 1.2 | 0.7 | 0.5 | 0.4 | 0.6 |
| Akiho | + | + | - | + | - | - |
| +: Discrimination band appeared in electrophoresis after PCR. -: Discrimination band did not appear in electrophoresis after PCR. | | | | | | |

Table 1-4

| Rice Variety Discrimination Band | S13 | T8 | T16 | WK9 |
|---|---|---|---|---|
| band length (kbp) | 1.8 | 0.9 | 1.6 | 1.6 |
| Koshihikari | - | - | - | - |
| Hitomebore | - | - | - | + |
| Hinohikari | - | - | - | + |
| Akitakomachi | - | - | - | + |
| Kirara 397 | + | + | + | + |
| Kinuhikari | - | + | + | + |
| Hoshinoyume | + | + | - | + |
| Haenuki | - | - | - | + |
| Mutsuhomare | - | + | + | - |
| Nipponbare | - | - | - | - |
| Sasanishiki | - | + | - | - |
| Tsugaruroman | - | + | - | + |
| Hanaechizen | - | + | + | + |
| Yumetsukushi | - | + | + | + |
| Hatsushimo | - | + | + | - |
| Asanohikari | - | + | + | + |
| Tsukinohikari | - | - | + | + |
| Aichinokaori | - | - | + | + |
| Matsuribare | - | - | + | + |
| Akiho | - | - | + | + |
| +: Discrimination band appeared in electrophoresis after PCR. -: Discrimination band did not appear in electrophoresis after PCR. | | | | |

From these bands, various STS primers were obtained.

(1) Discrimination band A7 (0.7 kbp) :

This band is given by the amplified DNAs from rice varieties "Koshihikari" and "Akitakomachi", but not specifically by those from "Nipponbare" and "Asanohikari". From the band A7, a pair of primers A7F30 (SEQ ID No. 5) and A7R30 (SEQ ID No. 6) were designed.
Next, a predetermined number of bases were deleted from the 3' -side of the primers to obtain STS primers for use in the invention, A7F19 (SEQ ID No. 7) and A7R16 (SEQ ID No. 8).

(2) Discrimination band B43 (0.9 kbp):

This band is given by the amplified DNAs from rice varieties "Koshihikari", "Hitomebore" and "Sasanishiki", but not by those from "Kirara 397" and "Asanohikari". From the band B43, a pair of primers B43F30 (SEQ ID No. 21) and B43R30 (SEQ ID No. 22) were designed.
Next, a predetermined number of bases were deleted from the 3'-side of the primers to obtain STS primers for use in the invention, B43F17 (SEQ ID No. 23) and B43R18 (SEQ ID No. 24).

(3) Discrimination band E30 (0.85 kbp):

This band is given by the amplified DNAs from rice varieties "Hitomebore" and "Mutsuhomare", but not by those from "Koshihikari" and "Akitakomachi". From the band E30, a pair of primers E30F30 (SEO ID No. 29) and E30R30 (SEQ ID No. 30) were designed.
Next, some bases were deleted from the primers to obtain STS primers for use in the invention, E30F28 (SEQ ID No. 31) and E30R24 (SEQ ID No. 32).

(4) Discrimination band J6 (0.9 kbp):

This band is given by the amplified DNAs from rice varieties "Koshihikari" and "Kirara 397", but not by those from "Sasanishiki". From the band J6, a pair of primers J6F30 (SEQ ID No. 49) and J6R30 (SEQ ID No. 50) were designed.
Next, some bases were deleted from the primers to obtain STS primers for use in the invention, J6F18 (SEQ ID No. 51) and J6R20 (SEQ ID No. 52).

(5) Discrimination band M2CG (1.2 kbp):

This band indicates addition of two bases to a 10-mer random primer, and this is given by the amplified DNAs from rice varieties "Hitomebore" and "Nipponbare" but not by those from "Koshihikari" and "Kinuhikari". From the band M2CG, a pair of primers M2CGF30 (SEQ ID No. 53) and M2CGR30 (SEQ ID No. 54) were designed.
Next, some bases were deleted from the primers to obtain STS primers for use in the invention, M2CGF16 (SEQ ID No. 55) and M2CCR15 (SEQ ID No. 56).

(6) Discrimination band S13 (1.8 kbp):

This band is given by the amplified DNAs from rice varieties "Kirara 397" and "Hoshinoyume", but not specifically by those from "Nipponbare" and "Asanohikari". From the, band S13, a pair of primers S13F30 (SEQ ID No. 73) and S13R30 (SEQ ID No. 74) were designed.
Next, some bases were deleted from the primers to obtain STS primers for use in the invention, S13F25 (SEQ ID No. 75) and S13R24 (SEQ ID No. 76).
From S13, S13F40 (SEQ ID No. 89) and S13R40 (SEQ ID No. 90) could be designed. However, S13F12 (SEQ ID No. 91) and S13R12 (SEQ ID No. 92) constructed by deleting some bases from these primers non-specifically bond to some other unintended DNA fragments and are mismatched with them, and, as a result, the band expression frequency not indicating the intended discrimination bands increases. After all, these primers are useless for rice variety discrimination in PCR with them.

(7) Discrimination band WK9 (1.6 kbp):

This band is given by the amplified DNAs from rice varieties "Hitomebore" and "Akitakomachi", but not by those from "Koshihikari" and "Sasanishiki" . From the band WK9, a pair of primers WK9F30 (SEQ ID No. 85) and WK9R30 (SEQ ID No. 86) were designed.
Next, some bases were deleted from the primers to obtain STS primers for use in the invention, WK9F20 (SEQ ID No. 87) and WK9R20(SEQ ID No. 88).
For obtaining the STS primers for use in the invention, some bases are deleted from the specifically designed primers as above. Briefly, a template DNA extracted from a rice sample is amplified, and a pair of primers are designed on the basis of the amplified DNA. Unnecessary bases are deleted from the thus-designed pair primers to give the intended STS primers of from 15 to 30 bases each.
Deleting the bases may be effected in any ordinary manner using suitable restriction endonuclease.
Concretely, from pairs of primers, A6F30 (SEQ ID No. 1) and A6R30 (SEQ ID No.2) ; A7F30 (SEQ ID No. 5) and A7R30 (SEQ ID No. 6) ; B1F30 (SEQ ID No. 9) and B1R30 (SEQ ID No. 10) ; B7F30 (SEQ ID No. 13) and B7R30 (SEQ ID No. 14) ; B18F30 (SEQ ID No. 17) and B18R30 (SEQ ID No. 18) ; B43F30 (SEQ ID No. 21) and F43R30 (SEQ ID No. 22) ; E22F30 (SEQ ID No. 25) and E22R30 (SEQ ID No. 26) ; E30F30 (SEQ ID No. 29) and E30R30 (SEQ ID No. 30) ; F6F30 (SEQ ID No. 33) and F6R30 (SEQ ID No. 34); G4F30 (SEQ ID No. 37) and G4R30 (SEQ ID No. 38) ; G22F30 (SEQ ID No. 41) and G22R30 (SEQ ID No. 42) ; G28F30 (SEQ ID No. 45) and G28R30 (SEQ ID No. 46) ; J6F30 (SEQ ID No. 49) and J6R30 (SEQ ID No. 50) ; M2CGF30 (SEQ ID No. 53) and M2CGR30 (SEQ ID No. 54) ; M11F30 (SEQ ID No. 57) and M11R30 (SEQ ID No. 58) ; P3F30 (SEQ ID No. 61) and P3R30 (SEQ ID No. 62); P5F30 (SEQ ID No. 65) and P5R30 (SEQ ID No. 66) ; Q16F30 (SEQ ID No. 69) and Q16R30 (SEQ ID No. 70) ; S13F30 (SEQ ID No. 73) and S13R30 (SEQ ID No. 74); T8F30 (SEQ ID No. 77) and T8R30 (SEQ ID No. 78); T16F30 (SEQ ID No. 81) and T16R30 (SEQ ID No. 82) ; WK9F30 (SEQ ID No. 85) and WK9R30 (SEQ ID No. 86), unnecessary bases are deleted according to the method mentioned above to give STS primers each composed of 15 to 30 bases. From the group of these STS primers, at least two pair primers are selected and used in the invention.

Accordingly, the STS primers for use in the invention are at least two or more oligonucleotides selected from a group of A6F21 (SEQ ID No. 3) and A6R22 (SEQ ID No. 4) ; B1F25 (SEQ ID No. 11) and B1R20 (SEQ ID No. 12) ; A7F22 (SEQ ID No. 15) and A7R17 (SEQ ID No. 16) ; B43F17 (SEQ ID No. 23) and B43R18 (SEQ ID No. 24) ; E30F28 (SEQ ID No. 31) and E30R24 (SEQ ID No. 32) ; F6F25 (SEQ ID No. 35) and F6R22 (SEQ ID No. 36) ; G4F18 (SEQ ID No. 39) and G4R24 (SEQ ID No. 40) ; G22F27 (SEQ ID No. 43) and G22R23 (SEQ ID No. 44) ; G28FI7 (SEQ ID No. 47) and G28R28 (SEQ ID No. 48) ; J6F18 (SEQ ID No. 51) and J6R20 (SEQ ID No. 52) ; M2CGF16 (SEQ ID No. 55) and M2CGR15 (SEQ ID No. 56) ; M11F20 (SEQ ID No. 59) and M11R20 (SEQ ID No. 60) ; P5F20 (SEQ ID No. 67) and P5R25 (SEO ID No . 68) ; S13F25 (SEQ ID No. 75) and S13R24 (SEQ ID No. 76) ; T16F24 (SEQ ID No. 83) and T16R26 (SEQ ID No. 84) ; WK9F20 (SEQ ID No. 87) and WK9R20 (SEQ ID No. 88).

Any other additional STS primers suitable to the invention may be designed in consideration of their capability for rice palatability evaluation and their melting temperature (Tm), and may be used in the invention.

Tm corresponds to the temperature at which the two strands of DNA are separated from each other. For the annealing temperature in PCR, in general, Tm or therearound of the primers used therein is suitable. In the method of the invention, in case of STS primers being used in combination, STS primers having a similar Tm are specifically selected and used, and the annealing temperature in PCR of DNA in the method is suitably so determined that it is near to Tm of the STS primers used. Therefore, in the method of the invention, the intended discrimination band to be given in a process where the STS primers are separately used can be obtained in one or a few PCR cycles.

Concretely, it is desirable that the difference between the average Tm of the STS primers to be used in the invention and Tm of each STS primer is not larger than 15°C ($\pm$ 15°C), and the annealing temperature in PCR also falls within the range.

If the annealing temperature in PCR is lower by 15°C or more than the mean Tm of the STS primers used, it is unfavorable since the varieties that should not give discrimination bands may give them through PCR. On the other hand, if the annealing temperature in PCR is higher by 15°C or more than the mean Tm of the STS primers used, it is also unfavorable since the discrimination band that should be given may disappear through PCR.

Suitably selected, the STS primers selectively bond, in PCR, to only the base sequence site of the DNA that gives a palatability evaluation band.

In the invention, one or more different types of such STS primers can be used.

Using di f ferent types of STS primers in the invention means that the selected different STS primers are used in one and the same reaction in PCR for variety discrimination. In the invention, a combination of suitably selected STS primers (as a primer set) may be used and a discrimination band corresponding to each primer used appears through electrophoresis. The invention has made it possible to use a combination of different STS primers for rice variety discrimination.

All the STS primers prepared could not be combined unconditionally. It is necessary to suitably combine the STS primers in a suitably selected blend ratio, taking the matters into consideration that the STS primers combined should not form primer dimers and the discrimination bands to appear should not overlap with each other. Suitably combining various STS primers and using the thus-combined STS primers removes the necessity of PCR for every primer, and makes it possible to accurately and simply discriminate many rice varieties from each other in only one PCR. To that effect, the invention significantly saves the labor, the time and the cost for rice variety discrimination.

The DNA-level rice palatability evaluation method for selecting palatable rice of the invention is described. The method comprises amplifying the DNA extracted from a rice plant, unhulled rice, unpolished rice, polished rice, boiled rice, rice cake or ground powder thereof through PCR in the presence of an STS primer, selecting a DNA band of close correlation with palatability evaluation from the resulting amplified DNA bands, and using it as a DNA marker for palatability evaluation to select palatable rice.

For rice palatability evaluation according to the method, the data that indicate the presence or absence of discrimination bands through PCR as in Table 1 are digitized. Concretely, they are binarized to the effect that the presence of discrimination bands in electrophoresis after PCR (+) is 1, and the absence thereof (-) is 0. For the rice palatability digitization, employable is a method of directly digitizing the results of organoleptic examination of the palatability of rice samples and the data of physicochemical examination of rice samples. Still another employable method is a method of digitizing the palatability of rice samples tried, for example, according to *Rice Palatability Ranking* (published by the Japan Association of Grain Inspection, 2001) to the effect that the rank of special A is 5 points, the rank of A is 4 points, the rank of A' is 3 points, the rank of B is 2 points and the rank of B' is 1 point. Further employable method is a method of digitizing the palatability of rice sample tried according to *Encyclopedia of Rice Varieties 2* (published by Rice Data Bank in 1999) in which the rank of five stars is 5 points, the rank of four stars is 4 points, the rank of three stars is 3 points, the rank of two stars is 2 points and the rank of one star is 1 point, and thus digitized, the data in this method may be combined with the above data in rice palatability ranking and the sum total of the data of each rice variety tested indicates the palatability evaluation thereof.

Thus digitized points of the data of rice sample, that is the presence or absence of discrimination bands through PCR effected in the presence of an STS primer or a random primer are established as explanatory variables; and the data thereof in any of palatability examination, organoleptic examination or physicochemical examination are established as response variables. These variables are processed through multivariate analysis such as multiple regression analysis to give a palatability estimation formula. From the palatability estimation formula, the multiple correlation function of each rice sample tested is calculated. Based on it, the rice tested is determined as to whether it is palatable rice or not.

The palatability estimation formula is generally obtained through multiple regression analysis. Apart from it, however, it may also be obtained through any other ordinary multivariate analysis, for example, through principal component analysis, cluster analysis, PLS analysis or neural network analysis.

For example, multiple regression analysis for rice palatability estimation in the invention may be effected as follows: A linear multiple regression model of the following formula is formed. In this, y indicates a response variable for palatability evaluation; $x_1$, $x_2$, $x_3$, ... $x_p$ indicate explanatory variables corresponding to the presence or absence of discrimination bands for from 1 to a number p primers; and $x_{11}$, $x_{21}$, $x_{31}$, ... $x_{p1}$ indicate explanatory variables to the response variables $y_1$, $y_2$, $y_3$, ... $y_p$ for from 1 to a number p individuals.

$$y_i = k + a_1 x_{1i} + a_2 x_{2i} + a_3 x_{3i} + \ldots + a_p x_{pi}$$

When the unknown constant term k and the regression coefficients $a_1$, $a_2$, $a_3$, ... $a_p$ are determined, then the estimated values of the response variables corresponding to any of the resulting explanatory variables $x_1$, $x_2$, $x_3$, ... $x_p$ can be calculated.

The regression coefficients $a_1$, $a_2$, $a_3$, ... $a_p$ shall be so determined that the square sum of the predicted error, the difference between the predicted value and the found value is minimized. Specifically, (p + 1)-dimensional simultaneous equations are written so as to minimize the value of the following formula, and their solutions for the regression coefficients $a_1$, $a_2$, $a_3$, ... $a_p$ are obtained. The constant term k is obtained by substituting the regression coefficients in the regression model formula.

**Predicted Error**

$$= \Sigma [y_i - (k + a_1 x_{1i} + a_2 x_{2i} + a_3 x_{3i} + \ldots + a_p x_{pi})]^2$$

in which i falls between 1 and p.

Next described is the method for selecting palatable rice of the invention. In the method for selecting palatable rice, the sample to be used for extracting its DNA is an embryo (germ)-free half grain of unhulled or unpolished rice. Concretely, an unhulled or unpolished grain of rice to be tested is halved with a razor or a cutter, and its genome DNA is extracted from an embryo (germ) -free half of the grain. Thus extracted, the DNA is subjected to PCR in which it serves as a template in the presence of various primers such as STS primers.

Next, the pattern of the DNA having been amplified through PCR is analyzed through electrophoresis. Through the process, the samples of unhulled or unpolished rice tested are screened to select those having given many discrimination DNA marker specific to palatable rice and few discrimination DNA marker specific to non-palatable rice. For the DNA marker for palatability evaluation, generally used are the DNA bands specific to the variety of palatable rice, or the DNA bands specific to the variety of non-palatable rice, but any other DNA bands than these may also be used.

After the palatable rice has been selected based on the DNA marker for palatability evaluation in the manner as above, the remaining half having an embryo (germ) of the unhulled or unpolished rice is allowed to germinate and grow into a rice plant to thereby selectively breed the thus-selected variety of palatable rice.

The embryo (germ)-having half grain of unhulled or unpolished rice is allowed to germinate and grow into a rice plant, and grains of the rice plant of the coming generation are harvested. This may be effected, for example, as follows: The embryo (germ) -having half grains of rice are processed with a 1 % sodium hypochlorite solution to sterilize their surfaces, then put on a medium having, for example, a composition mentioned below, and allowed to germinate at 30°C. These are germinated half grains of the selected palatable rice.

| Medium Composition (in one liter, pH 5.8): | |
|---|---|
| Agarose | 8.000 g |
| Ammonium sulfate | 0.463 g |
| Potassium nitrate | 2.830 g |
| Calcium chloride | 0.166 g |
| Magnesium sulfate | 0.185 g |
| Potassium dihydrogenphosphate | 0.400 g |
| Ferric sulfate | 0.278 g |
| Disodium EDTA | 0.373 g |
| Nicotinic acid | 0.250 mg |
| Vitamin B6 | 0.250 mg |
| itamin B1 | 0.500 mg |
| Glycine | 1.000 mg |

The germinated half grains are trans ferred into a phytotron, and cultured therein in a cycle at 28°C (in light) for 10 hours and 28°C (in dark) for 14 hours to thereby allow them to grow into green seedlings. Next, the seedlings are transplanted into an agricultural pot containing the same medium as above, allowed to grow therein, then transplanted into seedling compost and allowed to further grow in a phytotron for about 2 weeks. Thus having grown, the seedlings are then transplanted into a Wagner pot filled with potting compost, and cultivated in the phytotron for about 4 months to thereby allow them to put forth ears. These are further cultivated therein for about 40 days until their ears are ripened. Then, the ripened ears are harvested, threshed and hulled.

The hulled grains are polished and ground into powder, from which the DNA is extracted with CTAB method in the same manner as above. Serving as a template, the DNA is subjected to PCR in the presence of various primers such as STS primers and then to electrophoresis. Based on the presence or absence of DNA bands for palatability evaluation given by the amplified DNA in electrophoresis, the palatability estimation formula of the sample is written. From the formula, the sample is determined as to whether it is palatable rice or not.

The sample of unpolished rice may be polished in a laboratory rice mill, and may be tried for its palatability through organoleptic examination or physicochemical examination.

Apart from the process as above, half grains of unhulled or unpolished rice may also be used for breeding and selecting palatable rice, according to the process mentioned below. According to conventional method, grains of rice to be tested are dipped in warm water at about 40°C, then castrated, and pollinated with paternal pollen. Cultured in pots, the pollinated grains produce F1 seeds, and some of them are sampled. Not hulled or polished, each grain sample is halved into an embryo (germ) -free half grain and another half grain having an embryo. The embryo (germ) -free half grains are ground in a mortar, and processed with CTAB method to extract its DNA. This is a template DNA in PCR.

The template DNA is subjected to PCR with various primers such as STS primers, and the amplified DNA is analyzed through electrophoresis. The presence or absence of palatability evaluation DNA bands in electrophoresis is digitized to 1 or 0. Briefly, the data in electrophoresis are binarized to the effect that the presence of discrimination bands in electrophoresis after PCR (+) is 1, and the absence thereof (-) is 0.

The palatability digitization according to the above-mentioned methods in *Rice Palatability Ranking* (published by the Japan Association of Grain Inspection, 2001) and *Encyclopedia of Rice Varieties 2* (published by Rice Data Bank in 1999) as well as the data digitized from physicochemical examination or measurement of physical properties of rice mentioned hereinabove also applies to the process of rice palatability evaluation.

Thus digitized in point of the presence or absence of discrimination bands through PCR, the data of rice samples are explanatory variables; and the data thereof in palatability examination or physicochemical examination are response variables. These variables are processed through multivariate analysis such as multiple regression analysis to give a palatability estimation formula. From the palatability estimation formula, the multiple correlation function of each rice sample tested is calculated. Based on it, the rice tested is determined as to whether it is palatable rice or not. Briefly, the presence or absence of discrimination bands in electrophoresis after PCR is binarized and substituted in the palatability estimation formula to calculate the estimated palatability data. The seeds of which the estimated palatability data are higher or the seeds that are presumed to have suitable physical data of boiled rice from their template DNA are selected as palatable rice. For the seeds that are presumed to have suitable physical data of boiled rice from their template DNA, for example, those that are presumed to have larger data of stickiness/hardness are selected for Japanese, while those that are presumed to have larger data of hardness are for Indian.

Embryo (germ)-having half grains of the thus-selected seeds are sown, germinated, transplanted and grown according to the process mentioned above, and the ripened seeds are harvested from the grown plants. The thus-obtained F2 varieties of palatable rice are self-pollinated in an ordinary manner and established, whereby palatable rice can be selected and grown from half grains of unhulled or unpolished rice.

According to the method of the invention, it is possible to presume the physicochemical data of rice plants, rice seeds, rice and processed rice products and the physical data of boiled rice that have close correlation with the palatability of rice. A very small quantity of rice, for example, only one grain or a half grain of rice is enough for the method of palatability evaluation. Accordingly, the invention has made it possible to presume the palatability of rice by testing a half grain of unhulled or unpolished rice to thereby select palatable rice, and the remaining half grain may be allowed to germinate to grow into a rice plant. The seeds of the thus-grown rice plant may be processed according to the method of the invention for selecting palatable rice.

In addition, the method of the invention is applicable to various varieties of rice harvested in different countries in the world.

EXAMPLES

The invention is described in more detail with reference to the following Examples, which, however, are not intended to restrict the scope of the invention.

Example 1 (application of boiled rice PCR-based palatability estimation formula with response variables derived from organoleptic examination data, to unidentified samples):

Best twenty varieties of rice harvested in 1999 in Japan, "Koshihikari", "Hitomebore", "Hinohikari", "Akitakomachi" , "Kirara 397", "Kinuhikari", "Hoshinoyume", "Haenuki", "Mutsuhomare", "Nipponbare", "Sasanishiki", "Tsugaruroman", "Hanaechizen", "Yumetsukushi", "Hatsushimo", "Asanohikari", "Tsukinohikari", "Aichinokaori", "Matsuribare" and "Akiho" were tested, all unpolished. Using a laboratory rice mill, (Yamamoto Seisakusho's Ricepal VP31T), these were milled into polished rice to a yield of 90 %.

One grain of each polished rice sample was put into a 1.5 ml plastic tube (by Assist), and 35 $\mu$l of deionized water was added thereto. The tubes were stood on a stand for 1 hour, and the grains absorbed the water therein. With each tube opened, the sample grains were put in an electric rice cooker (Toshiba's RC-183, with 75 ml of deionized water put in the outer jacket), boiled for 15 minutes and then allowed to settle for 15 minutes therein to prepare boiled rice samples.

DNA was extracted from each sample as follows: Each one boiled rice grain was put in a microtube, to which was added 300 $\mu$l of 100 mM tris-HCl buffer (pH 8.0, containing 100 mM NaCl), and this was mashed in a pellet mixer (by Contes) . Next, 5 $\mu$l of heat-resistant amylase, $\alpha$-amylase (by Sigma, 790 Units/mg solid, 1 mg/ml) was added to it, and reacted at 60°C for 1 hour. Next, 5 $\mu$l of *Tritirachium* album-derived protease K (by Onko, 20 mg/ml) was added thereto, and reacted at 37°C for 2 hours.

After the enzymatic reaction, 1 ml of ethanol cooled to -20°C was added to each sample, and left at -20°C for 15 minutes Using a microcentrifuge, this was centrifuged (15000 G, 4°C, 15 minutes - the same shall apply hereinunder) to separate the precipitated residue. The residue was dissolved in 300 $\mu$l of TE (10 mM tris-HCl, pH 8.0, 1 mM EDTA), and 400 $\mu$l of phenol was added thereto. For DNA extraction, this was stirred in a rotary stirrer for 30 minutes.

Next, this was centrifuged (15000 G, 4°C, 15 minutes) to collect the supernatant, and PCI (phenol/chloroform/ isoamyl alcohol, 25/24/1) of the same amount as that of the supernatant was added thereto. This was kept as it was for 30 minutes for DNA extraction, and then centrifuged (15000 G, 4°C, 15 minutes) to collect the supernatant. 6 ml of 5 M NaCl was added thereto, and 400 $\mu$l of cold ethanol was added thereto. Then, this was centrifuged, and the resulting precipitate was washed twice with 70 % ethanol. The final precipitate was dissolved in 40 $\mu$l of 10-fold diluted TE to prepare a DNA sample solution.

Its PCR was carried out as follows:

The template DNA extracted from each boiled rice sample in the manner as above was amplified through PCR. The PCR composition was prepared by mixing 0.2 $\mu$l of Taq polymerase (by Takara Bio Inc., 5 U/$\mu$l), 2.5 $\mu$l of PCR buffer (12 mM tris-HCl, 60 mM KC1, pH 8.3), 2.0 $\mu$l of MgCl$_2$, 1 $\mu$l of the template DNA (200 ng/l $\mu$l) and 1 $\mu$l of dNTPs (100 $\mu$M). The STS primers for PCR are A6F21 and A6R22 (SEQ ID Nos. 3 and 4) of 0.4 $\mu$l each; A7F22 and A7R17 (SEQ ID Nos. 15 and 16) of 0.5 $\mu$l each; M11F20 and M11R20 (SEQ ID Nos. 59 and 60) of 0.6 $\mu$l each; S13F25 and S13R24 (SEQ ID Nos. 75 and 76) of 0.5 $\mu$l each; T16F24 and T16R26 (SEQ ID Nos. 83 and 84) of 0.4 $\mu$l each; J6F18 and J6R20 (SEQ ID Nos. 51 and 52) of 0.4 $\mu$l each; and WK9F20 and WK9R20 (SEQ ID Nos. 87 and 88) of 0.4 $\mu$l each, and these we're mixed with the PCR composition along with sterilized water to be 25.0 $\mu$l in total.

The reactor is PCR Thermal Cycler MP (by Takara Bio Inc.). In the reactor, the template DNA was subjected to 35-cycle PCR. One PCR cycle comprises denaturation at 94°C for 1 minute, annealing at 62°C for 1 minute and chain-extension at 72°C for 2 minutes.

The amplified DNA was then subjected to electrophoresis in Mupid II (by Cosmobio), in which it was allowed to migrate in 2 % agarose gel for 40 minutes, and stained with ethidium bromide to give a band pattern through exposure to UV light.

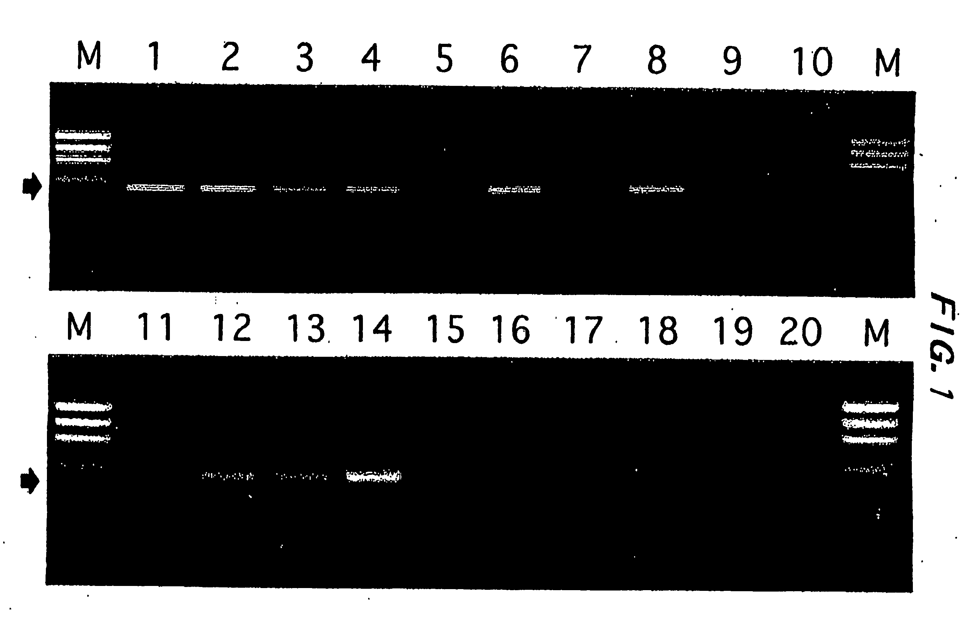

The palatability of each sample was digitized according to the method in *Rice Palatability Ranking* (published by the Japan Association of Grain Inspection, 2001). Concretely, the rank of special A is 5 points, the rank of A is 4 points, the rank of A' is 3 points, the rank of B is 2 points and the rank of B' is 1 point. Thus digitized, the data in the method were combined with the data digitized according to the method in *Encyclopedia of Rice Varieties 2* (published by Rice Data Bank in 1999) in which the rank of five stars is 5 points, the rank of four stars is 4 points, the rank of three stars is 3 points, the rank of two stars is 2 points and the rank of one star is 1 point. Thus combined, the sum total of the data of each sample tested indicates the palatability evaluation thereof. The band patterns of each sample detected in electrophoresis and the palatability evaluation data thereof are shown in Table 2 and Fig. 1.

In addition, the presence or absence of discrimination DNA bands in the band pattern of the DNA of each sample amplified through PCR as in Table 2 were binarized. Concretely, the presence of the discrimination DNA bands (+) is 1, and the absence thereof (-) is 0.

Further, the data thus obtained herein were processed through multiple regression analysis in which the binarized data indicating the presence or absence (1 or 0) of the discrimination bands with seven combinations of primers are the explanatory variables and the palatability evaluation data are the response variables. This gave the following palatability estimation formula:

$$\text{Palatability Estimation}$$
$$= 4.185 + 0.653 \times A6 + 1.068 \times A7 + 1.746 \times M11 + 1.42 \times S13$$
$$- 1.27 \times T16 - 0.355 \times WK9 + 1.81 \times J6 \qquad (1)$$

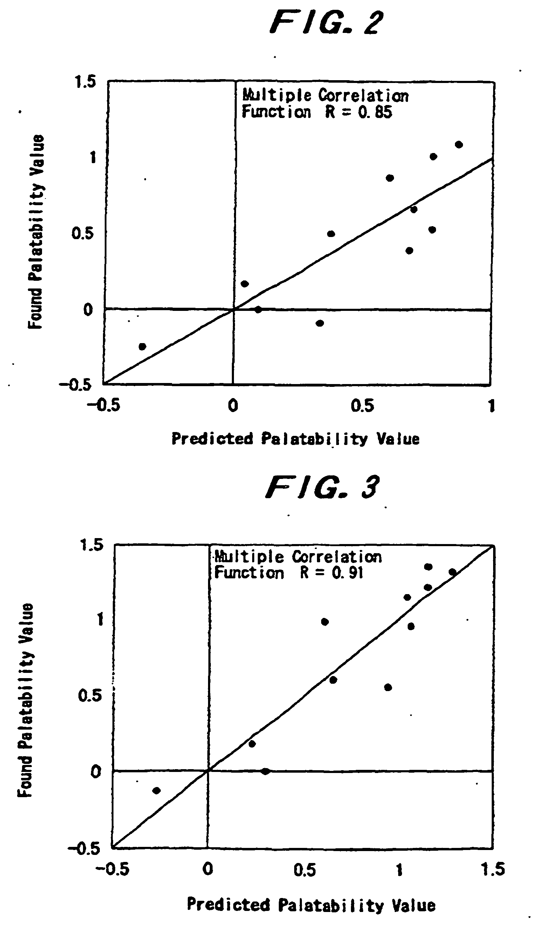

The applicability of the palatability estimation formula (1) to unidentified samples was investigated as follows: Eleven varieties of rice harvested in 1997 in Japan, 11 varieties in 1998 and 10 varieties in 1999 were tried and tested in the same manner as above and compared with each other in point of the value of palatability found through organoleptic examination and that predicted from the palatability estimation formula (1). Fig. 2 to Fig. 4 are graphs each showing a correlation between the found palatability value and the predicted palatability value.

As in these graphs indicating the data of the rice samples tried and tested herein, the multiple correlation function derived from the palatability estimation formula (1) is 0.85 for the samples of rice harvested in 1997, 0.91 for those in 1998 and 0.93 for those in 1999, and is all high. This confirms that the palatability estimation formula (1) is applicable to any other unidentified samples not used in completing the formula , irrespective of the date of the harvest time.

Table 2 - Popular Varieties of Rice, Their Data of Palatability Evaluated through Organoleptic Examination, and Primer-Dependent Discrimination Bands

| Varieties of rice | Data of Palatability Evaluated through Organoleptic Examination | | | | Primers Used | | | | | | |
|---|---|---|---|---|---|---|---|---|---|---|---|
| | reference point | harvested in 1997 | harvested in 1998 | harvested in 1999 | A6 | A7 | M11 | S13 | T16 | WK9 | J6 |
| Koshihikari | 10 | 1.09 | 1.32 | 0.37 | - | + | + | - | - | - | + |
| Hitomebore | 9 | 1.01 | 1.21 | 0.68 | - | + | + | - | - | + | + |
| Hinohikari | 8 | 0.53 | 1.35 | * | - | + | + | - | - | + | + |
| Akitakomachi | 8 | * | * | * | - | + | + | - | - | + | + |
| Kirara 397 | 7 | -0.09 | 0.98 | * | - | + | - | + | + | + | + |
| Kinuhikari | 8 | 0.87 | 0.55 | 0.21 | + | + | + | - | + | + | + |
| Hoshinoyume | 8 | 0.39 | 1.14 | 0.18 | - | + | - | + | - | + | + |
| Haenuki | 9 | * | * | * | + | + | + | - | - | + | + |
| Mutsuhomare | 6 | 0.17 | 0.18 | -0.62 | - | + | - | - | - | - | + |
| Nipponbare | 6 | 0.00 | 0.00 | 0.00 | - | + | + | - | + | - | + |
| Sasanishiki | 7 | 0.50 | 0.60 | 0.15 | - | + | + | - | - | - | - |
| Tsugaruroman | 8 | * | * | * | - | + | + | - | - | + | + |
| Hanaechizen | 6 | * | * | * | - | - | + | - | + | + | + |
| Yumetsukushi | 8 | * | * | * | - | + | + | - | - | + | + |
| Hatsushimo | 8 | 0.66 | 0.95 | 0.42 | + | + | + | - | + | - | + |
| Asanohikari | 5 | -0.25 | -0.13 | -0.94 | - | - | - | - | + | + | + |
| Tsukinohikari | 4 | * | * | -1.39 | - | - | - | - | + | + | + |
| Aichinokaori | 8 | * | * | * | + | + | + | - | + | + | + |
| Matsuribare | 6 | * | * | * | - | - | + | - | + | + | + |
| Akiho | 5 | * | * | * | - | + | - | - | + | + | + |

+: Discrimination band appeared in electrophoresis after PCR.
-: Discrimination band did not appear in electrophoresis after PCR.
* Not organoleptically tested.

Example 2 (practicality of polished rice PCR-based palatability estimation formula with response variables derived from organoleptic examination data):

This is the same as Example 1 except that powder of polished rice was sampled for PCR herein. Concretely, unpolished rice samples of the same best twenty varieties as in Example 1 were polished and ground into rice powder samples. A genome DNA was extracted from 6 g of each powder sample through CTAB treatment. Concretely, 6 ml of a 2 % CTAB solution (0.1 M tris-HCl, 2 mM disodium ethylenediaminetetraacetate (EDTA), 1.4 M NaCl, pH 8.0) at 70°C was added to the sample and stirred, and put into an incubator at 55°C, and 6 ml of the same CTAB (1 %) solution was added thereto. In that condition, the genome DNA was extracted from the sample for 30 minutes.

Next, chloroform/isoamyl alcohol (24/1) was added to the DNA extract, stirred and centrifuged. A DNA precipitant (1 % CTAB solution, 20 mM tris-HCl, 10 mM EDTA, pH 8.0) was added to the resulting supernatant, and left at 4°C overnight to precipitate the DNA. Next, this was centrifuged, and the resulting DNA precipitate was extracted with 1 M NaCl. The DNA extract was washed with isopropyl alcohol and ethanol, then precipitated, and dissolved in 200 $\mu$l of a TE buffer (10 mM tris-HCl, 1 mM EDTA, pH 8.0) to prepare a DNA sample solution.

The template DNA extracted from each polished rice sample was subjected to PCR with five different types of STS primers, B1, F6, G28, P5 and T16. The PCR composition was prepared by mixing 0.2 $\mu$l of Taq polymerase (by Takara Bio Inc., 5 U/$\mu$l), 2.5 $\mu$l of PCR buffer (12 mM tris-HCl, 60 mM KCl, pH 8.3), 2.0 $\mu$l of $MgCl_2$, 1 $\mu$l of the template DNA (200 ng/l $\mu$l) and 1 $\mu$l of dNTPs (100 $\mu$M). The STS primers for PCR are B1F25 (SEQ ID No. 11) and B1R20 (SEQ ID No. 12) of 0.2 $\mu$l each; F6F25 and F6R22 (SEQ ID Nos. 35 and 36) of 0.4 $\mu$l each; G28F17 and G28R28 (SEQ ID Nos. 47 and 48) of 0.2 $\mu$l each; P5F20 and P5R25 (SEQ ID Nos. 67 and 68) of 0.3 $\mu$l each; and T16F24 and T16R26 (SEQ ID Nos. 83 and 84) of 0.3 $\mu$l each, and these were mixed with the PCR composition along with sterilized water to be 25.0 $\mu$l in total. The PCR condition is the same as in Example 1.

The amplified DNA was then subjected to electrophoresis in Mupid II (by Cosmobio), in which it was allowed to migrate in 2 % agarose gel for 40 minutes, and stained with ethidium bromide to give a band pattern through exposure to UV light.

On the other hand, the rice samples were organoleptically tested for digitizing their palatability. The organoleptic test is as follows: 600 g of each polished rice sample was washed with water. Pure water of 1.3 times (by weight) the sample was added thereto, and it was allowed to absorb the pure water for 1 hour. This was boiled in a rice cooker (Toshiba's RC-183). After the rice cooker was automatically switched off, it was left as it was for 15 minute to settle the boiled rice therein. Kept covered with its lid, this was further left as it was for 1 hour. Then, this was opened, and the boiled rice therein was gently stirred with a rice scoop. The boiled rice was put into small cups each on a dish, and tried by panelists.

The number of the panelists was 24. The details of the organoleptic test of the rice samples are as follows: "Nipponbare" is the reference for palatability evaluation of the samples, and this is given a point of 0. The other samples were compared with the reference for the comprehensive palatability evaluation thereof. The samples that tasted definitely very good at the first mouthful are given a point of +3; those that tasted relatively good at the first mouthful, though not definitely, are given a point +2; those that were not definite at the first mouthful but tasted good a little at the second mouthful are given a point +1; those that tasted definitely not good at the first mouthful are given a point of -3; those that tasted not good a little at the first mouthful, though not definitely, are given a point of -2; and those that were not definite at the first mouthful but tasted not good a little at the second mouthful are given a point -1. The points given by all the panelists to each sample were averaged to evaluate the palatability of the individual samples. The results of the band patterns of the samples having appeared in electrophoresis, and the results of the organoleptic palatability evaluation are given in Table 3.

In addition, the presence or absence of discrimination DNA bands in the band pattern of the DNA of each sample amplified through PCR as in Table 3 was binarized. Concretely, the presence of the discrimination DNA bands (+) is 1, and the absence thereof (-) is 0.

Further, the data thus obtained herein were processed through multiple regression analysis in which the binarized data indicating the presence or absence (1 or 0) of the discrimination bands with five combinations of primers are the explanatory variables and the organoleptic palatability evaluation data of the 11 varieties of rice harvested in 1998 and tested herein are the response variables. This gave the following palatability estimation formula (2). The multiple correlation function derived from the palatability estimation formula (2) for the tested samples is 0.87.

## Palatability Estimation

$$= 0.777 - 0.359 \times B1 - 0.288 \times F6 + 0.135 \times G28 + 0.519 \times P5 - 0.215 \times T16 \qquad (2)$$

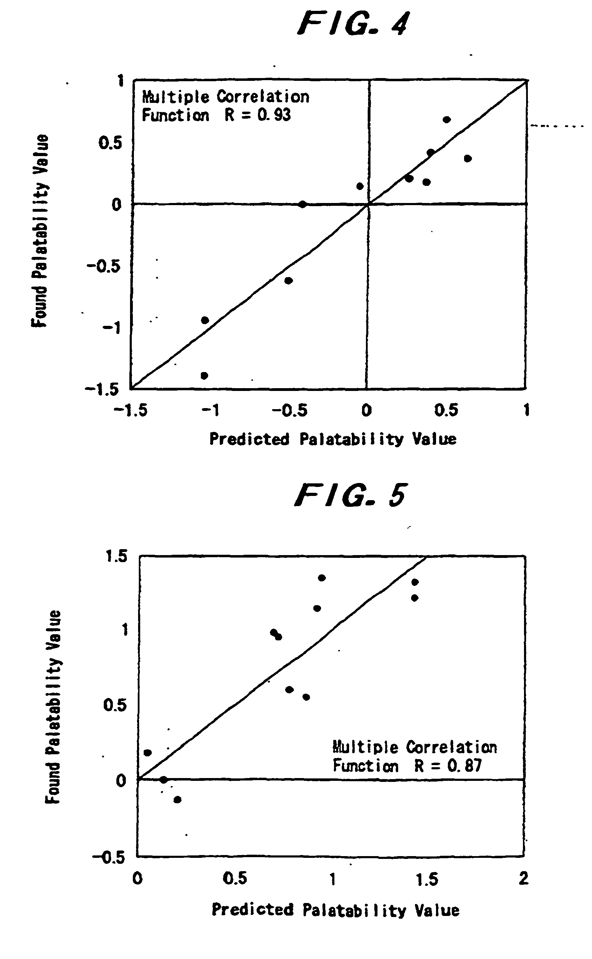

The compatibility of the palatability estimation formula (2) with the organoleptic examination data of 12 varieties of rice harvested in 1999, "Koshihikari", "Hitomebore", "Hinohikari", "Sasanishiki", "Hoshinoyume", "Kirara 397", "Mutsuhomare", "Asanohikari", "Hatsushimo", "Kinuhikari", "Nipponbare" and "Tsukinohikari" was investigated as follows: The palatability values of these 12 varieties of rice in 1999 that had been tested and found through organoleptic examination as above were compared with those thereof predicted from the palatability estimation formula (2). Fig. 5 is a graph showing a correlation between the found palatability value and the predicted palatability value.

As is obvious from the above, the multiple correlation function for the 12 varieties of rice in 1999 is 0.85. This confirms that the PCR-based palatability estimation formula in the invention is applicable to unidentified varieties of rice harvested in the next year.

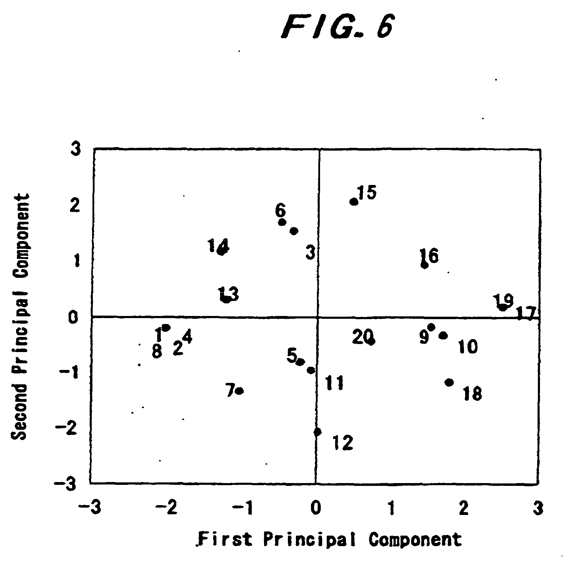

In addition, the rice samples were analyzed for the principal components thereof, based on the digitized PCR data as variables. Fig. 6 is a graph showing the analyzed data of the principal components plotted therein. In Fig. 6, the numerical value of plots corresponds to the best twenty varieties of rice harvested in 1999 in Japan, that is to say, 1 denotes "Koshihikari", 2 denotes "Hitomebore", 3 denotes "Hinohikari", 4 denotes "Akitakomachi", 5 denotes "Kirara 397", 6 denotes "Kinuhikari", 7 denotes "Hoshinoyume", 8 denotes "Haenuki" , 9 denotes "Mutsuhomare" , 10 denotes "Nipponbare", 11 denotes "Sasanishiki" , 12 denotes "Tsugaruroman", 13 denotes "Hanaechizen", 14 denotes "Yumetsukushi", 15 denotes "Hatsushimo", 16 denotes "Asanohikari", 17 denotes "Tsukinohikari", 18 denotes "Aichinokaori", 19 denotes "Matsuribare", and 20 denotes "Akiho", respectively.

As in Fig. 6, the plots indicate the individual rice samples analyzed. This supports the applicability of the PCR-based multivariate analysis in the invention to classification of the varieties of rice based on organoleptic examination.

Table 3 - Organoleptic Examination Data and PCR Data

| | Data of Palatability Evaluated through Organoleptic Examination | | Primers used in PCR, and Estimated Value of Palatability | | | | | |
|---|---|---|---|---|---|---|---|---|
| | harvested in 1998 | harvested in 1999 | B1 | F6 | G28 | P5 | T16 | Estimated Value of Palatability |
| Koshihikari | 1.32 | 0.37 | - | - | + | + | - | 1.43 |
| Hitomebore | 1.21 | 0.68 | - | - | + | + | - | 1.43 |
| Hinohikari | 1.35 | * | + | - | - | + | - | 0.94 |
| Kirara 397 | 0.98 | * | - | - | + | - | + | 0.70 |
| Kinuhikari | 0.55 | 0.21 | + | - | + | + | + | 0.86 |
| Hoshinoyume | 1.14 | 0.18 | - | - | + | - | - | 0.91 |
| Mutsuhomare | 0.18 | -0.62 | + | + | + | - | + | 0.05 |
| Nipponbare | 0.00 | 0.00 | + | + | - | - | - | 0.13 |
| Sasanishiki | 0.60 | 0.15 | - | - | - | - | - | 0.78 |
| Hatsushimo | 0.95 | 0.42 | + | - | - | + | + | 0.72 |
| Asanohikari | -0.13 | -0.94 | + | - | - | - | + | 0.20 |
| Tsukinohikari | * | -0.39 | + | + | - | - | + | 0.09 |
| +: Discrimination band appeared in electrophoresis after PCR. -: Discrimination band did not appear in electrophoresis after PCR. * Not organoleptically tested. | | | | | | | | |

Example 3 (practicality of boiled rice PCR-based palatability estimation formula with response variables derived from organoleptic examination data):

Eleven varieties of rice, "Koshihikari", "Hitomebore", "Hinohikari", "Sasanishiki", "Hoshinoyume", "Kirara 397", "Mutsuhomare", "Asanohikari", "Hatsushimo", "Kinuhikari" and "Nipponbare" harvested in 1997 and 1998, totaling 22 samples, were tested in the same manner as in Example 1. Briefly, the boiled rice samples were organoleptically tested, and DNA extracted from each boiled rice sample was used as the template DNA and was subjected to PCR.

In PCR, herein used were five different types of STS primers, 81, F6, G28, M11 and P5. The PCR composition was prepared by mixing 0.2 $\mu$l of Taq polymerase (by Takara Bio Inc., 5 U/$\mu$l), 2.5 $\mu$l of PCR buffer (12 mM tris-HCl, 60 mM KCl, pH 8.3), 2.0 $\mu$l of MgCl$_2$, 1 $\mu$l of the template DNA (200 ng/l $\mu$l) and 1 $\mu$l of dNTPs (100 $\mu$M). The STS primers for PCR are B1F25 (SEQ ID No. 11) and B1R20 (SEQ ID No. 12) of 0.2 $\mu$l each; F6F25 and F6R22 (SEQ ID Nos. 35 and 36) of 0.4 $\mu$l each; G28F17 and G28R28 (SEQ ID Nos. 47 and 48) of 0.2 $\mu$l each; M11F20 and M11R20 (SEQ ID Nos. 59 and 60) of 0.2 $\mu$l each; and P5F20 and P5R25 (SEQ ID Nos. 67 and 68) of 0.2 $\mu$l each, and these were mixed with the PCR composition along with sterilized water to be 25.0 $\mu$l in total. The PCR condition is the same as in Example 1.

The amplified DNA was then subjected to electrophoresis in Mupid II (by Cosmobio), in which it was allowed to migrate in 2 % agarose gel for 40 minutes, and stained with ethidium bromide to give a band pattern through exposure to UV light.

On the other hand, the rice samples were organoleptically tested for digitizing their palatability, in the same manner as in Example 2. The results of the band patterns of the samples having appeared in electrophoresis, and the results of the organoleptic palatability evaluation are given in Table 4.

In addition, the presence or absence of discrimination DNA bands in the band pattern of the DNA of each sample amplified through PCR was binarized. Concretely, the presence of the discrimination DNA bands (+) is 1, and the absence thereof (-) is 0.

Further, the data thus obtained herein were processed through multiple regression analysis in which the binarized data indicating the presence or absence (1 or 0) of the discrimination bands with five combinations of primers are the explanatory variables and the organoleptic palatability evaluation data of the 11 varieties of rice harvested in 1998 and tested herein are the response variables. This gave the following palatability estimation formula (3). The multiple correlation function derived from the palatability estimation formula (3) for the tested samples is 0.80.

$$\text{Palatability Estimation}$$
$$= 0.745 - 0.501 \times B1 - 0.2181 \times F6 + 0.1273 \times G28 - 0.1454 \times$$
$$M11 + 0.6 \times P5 \qquad\qquad (3)$$

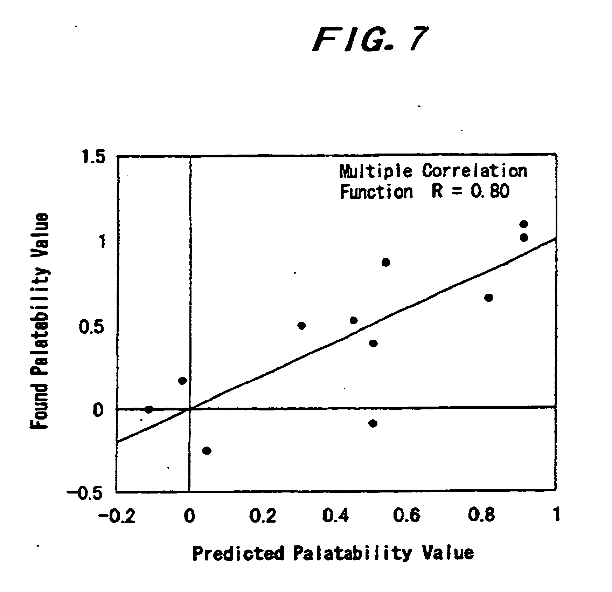

The palatability estimation formula (3) was applied to the unidentified samples of rice harvested in 1997, and it gave a multiple correlation function for rice in 1997 of 0.80 (Fig. 7). This confirms that the palatability estimation formula in the invention well applies to rice samples produced in different crop year.

Table 4 - Organoleptic Examination Data and PCR Data

|  | Organoleptic Examination Data | | Primers used in PCR | | | | |
|---|---|---|---|---|---|---|---|
|  | harvested in 1997 | harvested in 1998 | B1 | F6 | G28 | P5 | M11 |
| Koshihikari | 1.09 | 1.32 | - | - | + | + | + |
| Hitomebore | 1.01 | 1.21 | - | - | + | + | + |
| Hinohikari | 0.53 | 1.35 | + | - | - | + | + |
| Kirara 397 | -0.09 | 0.98 | - | - | + | - | - |
| Kinuhikari | 0.87 | 0.55 | + | - | + | + | + |
| Hoshinoyume | 0.39 | 1.14 | - | - | + | - | - |
| Mutsuhomare | 0.17 | 0.18 | + | + | + | - | - |
| Nipponbare | 0.00 | 0.00 | + | + | - | - | - |
| Sasanishiki | 0.50 | 0.60 | - | - | - | - | + |

(continued)

| | Organoleptic Examination Data | | Primers used in PCR | | | | |
|---|---|---|---|---|---|---|---|
| | harvested in 1997 | harvested in 1998 | B1 | F6 | G28 | P5 | M11 |
| Hatsushimo | 0.66 | 0.95 | + | - | - | + | + |
| Asanohikari | -0.25 | -0.13 | + | - | - | - | - |
| +: Discrimination band appeared in electrophoresis after PCR.<br>-: Discrimination band did not appear in electrophoresis after PCR.<br>* Not organoleptically tested. | | | | | | | |

Example 4 (practicality of polished rice PCR-based palatability estimation formula with response variables derived from physical data of boiled rice):

This is to investigate the practicality of the invention for rice palatability evaluation and estimation, based on the physical data of boiled rice that are the basis of the palatability of rice.

Twelve varieties of rice, "Koshihikari", "Hitomebore", "Hinohikari", "Kirara 397", "Kinuhikari", "Hoshinoyume", "Mutsuhomare", "Nipponbare", "Sasanishiki", "Hatsushimo", "Asanohikari" and "Tsukinohikari" harvested in 1998, and ten varieties of rice, "Koshihikari", "Hitomebore", "Kinuhikari", "Hoshinoyume", "Mutsuhomare", "Nipponbare", "Sasanishiki", "Hatsushimo", "Asanohikari" and "Tsukinohikari" harvested in 1999 were tested in the same manner as in Example 2. Briefly, a DNA was extracted from each polished rice powder sample, purified, and subjected to PCR with various STS primers and to electrophoresis to see the presence or absence of discrimination bands from the amplified DNA.

The PCR in this Example is the same as in Example 2, except that five different types of STS primers, B43F17 and B43R18 (SEQ ID Nos. 23 and 24) of 0.1 $\mu$l each; G22F27 and G22R23 (SEQ ID Nos. 43 and 44) of 0.3 $\mu$l each; P5F20 and P5R25 (SEQ ID Nos. 67 and 68) of 0.3 $\mu$l each; G4F18 and G4R24 (SEQ ID Nos. 39 and 40) of 0.2 $\mu$l each; and M2CGF16 and M2CGR15 (SEQ ID Nos. 55 and 56) of 0.2 $\mu$l each, were used herein.

The amplified DNA was subjected to electrophoresis in Mupid II (by Cosmobio), in which it was allowed to migrate in 2 % agarose gel for 40 minutes, and stained with ethidium bromide to give a band pattern through exposure to UV light.

Physical properties of boiled rice were measured as follows: 10 g of each polished rice sample was put into a small cup, 16 ml of pure water was added thereto, and it was allowed to absorb the pure water for 1 hour. Five small cups each with the sample rice were put into a rice cooker (Toshiba's RC-183), and boiled with 75 ml of pure water being around the cups therein . After the rice cooker was automatically switched off, it was left as it was for 15 minute to settle the boiled rice therein. This was further left at 25°C for 2 hours.

Using a tensile compression tester, Tensipresser, Myboy (by Takemoto Electric), 20 grains of each boiled rice sample were subjected to a low compression test of compressing them by 25 % of their thickness to measure the amount (L3) of the boiled rice grain stuck to the tester surface. The results are given in Table 5.

In addition, the presence or absence of discrimination DNA bands in the band pattern of the DNA of each sample amplified through PCR were binarized. Concretely, the presence of the discrimination DNA bands (+) is 1, and the absence thereof (-) is 0.

Further, the data thus obtained herein were processed through multiple regression analysis in which the binarized data indicating the presence or absence (1 or 0) of the discrimination bands with five combinations of primers are the explanatory variables and the measured physical data (L3) of the samples of rice in 1998 are the response variables. This gave the following physical data estimation formula (palatability estimation formula) (4). The multiple correlation function derived from this physical data estimation formula for the rice samples tested is 0.99.

.

$$\text{Palatability Estimation}$$

$$= 1.093 - 0.0858 \times \text{B43} - 0.197 \times \text{G4} + 0.235 \times \text{G22} + 0.0391 \times$$

$$\text{M2CG} + 0.100 \times \text{P5} \qquad\qquad (4)$$

Table 5 - Physical Data of Boiled Rice, and PCR Data

|  | Physical Data of Boiled Rice | | Primers Used in PCR | | | | |
|  | harvested in 1998 | harvested in 1999 | B43 | G4 | G22 | M2CG | P5 |
| --- | --- | --- | --- | --- | --- | --- | --- |
| Koshihikari | 1.44 | 1.50 | + | - | + | - | + |
| Hitomebore | 1.39 | 1.70 | + | - | + | + | + |
| Hinohikari | 1.48 | - | - | - | + | + | + |
| Kirara 397 | 1.17 | - | - | + | + | + | - |
| Kinuhikari | 1.19 | 1.23 | - | - | - | - | + |
| Hoshinoyume | 1.35 | 1.43 | - | - | - | + | - |
| Mutsuhomare | 1.07 | 1.21 | + | - | - | + | - |
| Nipponbare | 1.01 | 1.15 | + | - | - | + | - |
| Sasanishiki | 1.26 | 1.45 | + | - | + | - | - |
| Hatsushimo | 1.15 | 1.42 | + | - | - | + | + |
| Asanohikari | 1.10 | 1.08 | - | - | - | - | - |
| Tsukinohikari | 1.05 | 1.01 | - | - | - | + | - |
| +: Discrimination band appeared in electrophoresis after PCR.<br>-: Discrimination band did not appear in electrophoresis after PCR.<br>Physical data: in terms of the amount L3 (mm) of the boiled rice grain stuck to the surface of the tester, Tensipresser in low compression test. -: not examined. | | | | | | | |

The formula 4 resulting from multiple regression analysis was applied to next crop year samples of rice in 1999, and the multiple correlation function for the unidentified samples was 0.88. This supports the practicality of the DNA analysis-based physical data estimation formula in rice palatability evaluation.

Example 5 (preparation of polished rice PCR-based palatability estimation formula with response variables derived from physical data of boiled rice in different countries):

This is to investigate the application of the invention for rice palatability evaluation and estimation, based on the physical data of boiled rice in different countries. The physical data of boiled rice are the basis of the palatability of rice.
Fifteen varieties of rice harvested in different countries, "Koshihikari" in Japan, "Ilpum" in Korea, "Fuj ihikari" in China, "Erio" in Italy, "Basmati" and "Bangana" in India, "Tamsoan" and "C70" in Vietnam, "Oryza glaberrima (CG-14)" and "WAB450106" in Nigeria, "Amaroo" and "Pelde" in Australia, "Khao Dawk Mali" in Thailand, and "Nishiki" and "Bengal" in USA, were tested in the same manner as in Example 2. Briefly, DNAs were extracted from each polished rice powder sample, purified, and subjected to PCR with various STS primers and to electrophoresis to see the presence or absence of discrimination bands from the amplified DNA.
Eight different types of STS primers, A6F21 and A6R22 (SEQ ID Nos. 3 and 4) of 0.4 $\mu$l each; E30F28 and E30R24 (SEQ ID Nos. 31 and 32) of 0.6 $\mu$l each; F6F25 and F6R22 (SEQ ID Nos. 35 and 36) of 0.4 $\mu$l each; G4F18 and G4R24 (SEQ ID Nos. 39 and 40) of 0.5 $\mu$l each; J6F18 and J6R20 (SEQ ID Nos. 51 and 52) of 0.5 $\mu$l each; M2CGF16 and M2CGR15 (SEQ ID Nos. 55 and 56) of 0.5 $\mu$l each; S13F25 and S13R24 (SEQ ID Nos. 75 and 76) of 0.5 $\mu$l each; and WK9F20 and WK9R20 (SEQ ID Nos. 87 and 88) of 0.4 $\mu$l each, were used herein. The PCR condition is the same as in Example 2.
The DNAs amplified through PCR were subjected to electrophoresis in Mupid II (by Cosmobio), in which they were allowed to migrate in 2 % agarose gel for 40 minutes, and stained with ethidium bromide to give a band pattern through exposure to UV light.
Physical properties of boiled rice were measured as follows: 10 g of each polished rice sample was put into a small cup, 16 ml of pure water was added thereto, and it was allowed to absorb the pure water for 1 hour. Five small cups each with the sample rice were put into a rice cooker (Toshiba's RC-183), and boiled with 75 ml of pure water being around the cups therein. After the rice cooker was automatically switched off, it was left as it was for 15 minute to settle the boiled rice therein. This was further left at 25°C for 2 hours.
Using a tensile compression tester, Texturometer (by Zenkensha), 3 grains of each boiled rice sample were tested to measure their hardness (H1). In one test, 5 set of 3-grain samples were used in total to test one rice sample.

In addition, the presence or absence of discrimination DNA bands in the band pattern of the DNA of each sample amplified through PCR were binarized. Concretely, the presence of the discrimination DNA bands (+) is 1, and the absence thereof (-) is 0.

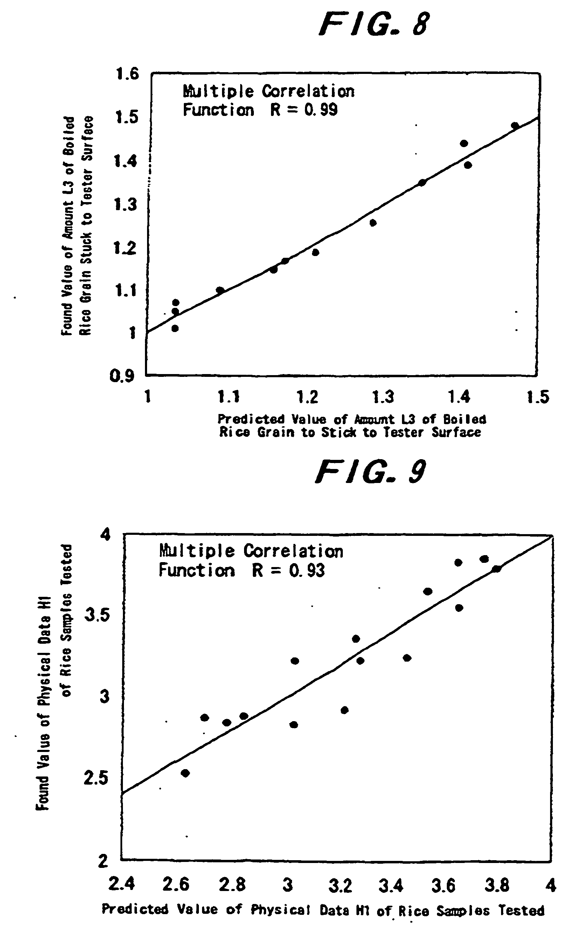

Further, the data thus obtained herein were processed through multiple regression analysis in which the binarized data indicating the presence or absence (1 or 0) of the discrimination bands with eight combinations of primers are the explanatory variables and the measured physical data (HI) of the rice samples are the response variables. This gave the following physical data estimation formula (palatability estimation formula) (5). In Fig. 9, the physical data actually obtained through physical examination, or that is, the found physical data of the rice samples tested are compared with the predicted physical data thereof estimated from the physical data estimation formula (5). Fig. 9 shows a correlation between the found value of physical data of the rice samples tested and the predicted value thereof. The multiple correlation function derived from this physical data estimation formula for the rice samples tested is 0.93.

$$y = 2.246 + 0.632 \times WK9 + 0.514 \times E30 - 0.703 \times A6 + 1.011 \times M2CG - 0.578$$
$$\times S13 + 0.386 \times J6 - 0.044 \times G4 + 0.38 \times F6 \qquad (5)$$

Table 6 - Physical Data of Boiled Rice, and PCR Data

| | Physical Data | Primers used in PCR | | | | | | | |
|---|---|---|---|---|---|---|---|---|---|
| | Hardness | A6 | E30 | F6 | G4 | J6 | M2CG | S13 | WK9 |
| Koshihikari | 2.53 | - | - | - | - | + | - | - | - |
| Ilpum | 2.88 | + | + | - | + | - | - | - | + |
| Fujihikari | 2.84 | + | + | + | + | + | - | - | - |
| Bengal | 3.22 | + | + | - | + | - | + | - | - |
| Tamsoan | 2.83 | + | + | - | + | + | + | + | - |
| C70 | 3.83 | - | - | - | + | - | + | + | + |
| Khao Dawk Mali | 3.36 | - | - | - | - | - | + | - | - |
| Erio | 3.79 | + | + | + | + | + | + | + | - |
| Amaroo | 2.87 | + | - | + | + | + | + | + | - |
| Pelde | 3.24 | + | + | + | + | - | + | + | + |
| Oryza glaberrima | 3.55 | + | + | - | + | + | + | + | + |
| Basmati | 3.85 | + | + | + | + | - | + | - | + |
| Bangana | 2.92 | - | - | - | + | - | + | - | - |
| WAB450106 | 3.65 | - | + | - | + | + | + | + | - |
| Nishiki | 3.22 | + | - | - | + | + | + | - | - |
| +: Discrimination band appeared in electrophoresis after PCR.<br>-: Discrimination band did not appear in electrophoresis after PCR.<br>Physical data: in terms of the hardness (H1, kgf) of boiled rice measured with Texturometer. | | | | | | | | | |

The results confirm that the multivariate analysis in the invention in which the explanatory variables are from the digitization of the presence or absence of the discrimination. DNA bands in PCR with STS primers is applicable to different varieties of rice harvested in various countries in the world for predicting the physical properties of different types of boiled rice.

Example 6 (preparation of PCR-based palatability estimation formula with response variables derived from physical data of boiled rice in different countries):

This is to investigate the application of the invention for rice palatability evaluation and estimation, based on the physical data of boiled rice in different countries. The physical data of boiled rice are the basis of the palatability of rice.

Eleven varieties of rice harvested in different countries, "Koshihikari" in Japan, "Fujihikari" in China, "Erio", "Ceremio", "Thaibonnet" and "Alborio" in Italy, "Oryza glaberrima (CG-14)", "WAB450106", "WAB9611", "WITA7" and "Cisdane" in Nigeria, were tested in the same manner as in Example 2. Briefly, DNAs were extracted from each polished rice powder sample, purified, and subj ected to PCR with various STS primers and to electrophoresis to see the presence or absence of discrimination bands from the amplified DNA.

Seven different types of STS primers, A6F21 and A6R22 (SEQ ID Nos. 3 and 4) of 0.4 $\mu$l each; B1F25 and B1R20 (SEQ ID Nos. 11 and 12) of 0.4 $\mu$l each; E30F28 and E30R24 (SEQ ID Nos. 31 and 32) of 0.6 $\mu$l each; F6F25 and F6R22 (SEQ ID Nos. 35 and 36) of 0.4 $\mu$l each; J6F18 and J6R20 (SEQ ID Nos. 51 and 52) of 0.5 $\mu$l each; S13F25 and S13R24. (SEQ ID Nos. 75 and 76) of 0.5 $\mu$l each; and WK9F20 and WK9R20 (SEQ ID Nos. 87 and 88) of 0.4 $\mu$l each, were used herein.

The DNAs amplified through PCR were subjected to electrophoresis in Mupid II (by Cosmobio), in which they were allowed to migrate in 2% agarose gel for 40 minutes, and stained with ethidium bromide to give a band pattern through exposure to UV light.

10 g of each polished rice sample was put into a small cup, 16 ml of pure water was added thereto, and it was allowed to absorb the pure water for 1 hour. Five small cups each with the sample rice were put into a rice cooker (Toshiba's RC-183), and boiled with 75 ml of pure water being around the cups therein. After the rice cooker was automatically switched off, it was left as it was for 15 minute to settle the boiled rice therein. This was further left at 25°C for 2 hours.

Using a tensile compression tester, Tensipresser, Myboy (by Takemoto Electric), 20 grains of each boiled rice sample were subjected to a low compression test of compressing them by 25 % of their thickness to measure the amount (L3) of the boiled rice grain stuck to the tester surface.

In addition, the presence or absence of discrimination DNA bands in the band pattern of the DNA of each sample amplified through PCR were binarized. Concretely, the presence of the discrimination DNA bands (+) is 1, and the absence thereof (-) is 0.

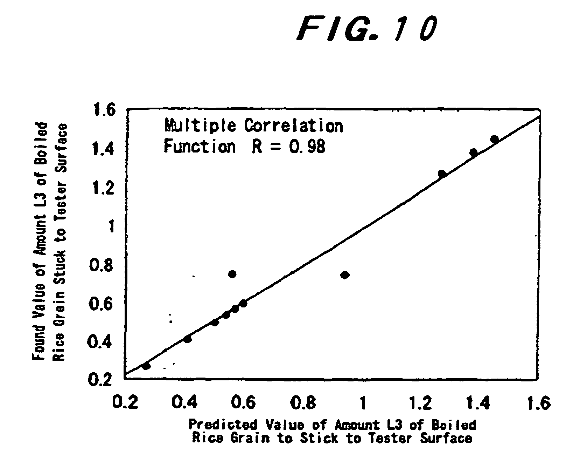

Further, the data thus obtained herein were processed through multiple regression analysis in which the binarized data indicating the presence or absence (1 or 0) of the discrimination bands with seven combinations of primers are the explanatory variables and the measured physical data (L3) of the rice samples are the response variables. This gave the following physical data estimation formula (palatability estimation formula) (6) . In Fig. 10, thephysicaldataactually obtained through physical examination, or that is, the found physical data of the rice samples tested are compared with the predicted physical data thereof estimated from the physical data estimation formula (6). Fig. 10 shows a correlation between the found value of physical data of the rice samples tested and the predicted value thereof. The multiple correlation function derived from this physical data estimation formula for the rice samples tested is 0.98.

$$y = 2.294 - 0.789 \times WK9 - 0.73 \times E30 - 0.82 \times A6 - 1.84 \times B1$$
$$+ 1.66 \times S13 - 0.845 \times J6 + 2.68 \times F6 \qquad (6)$$

Table 7 - Physical Data of Boiled Rice, and PCR Data

|  | Physical Data | Primers Used in PCR | | | | | | |
|---|---|---|---|---|---|---|---|---|
|  | amount of rice stuck to tester | A6 | E30 | F6 | B1 | J6 | WK9 | S13 |
| Koshihikari | 1.45 | - | - | - | - | + | - | - |
| Fujihikari | 0.94 | + | + | + | + | + | - | - |
| Erio | 0.56 | + | + | + | + | + | - | + |
| Ceremio | 1.27 | - | - | - | + | + | - | + |
| Thaibonnet | 0.60 | - | + | - | + | - | + | + |
| Alborio | 1.38 | - | + | - | + | - | - | + |

(continued)

| | Physical Data | Primers Used in PCR | | | | | | |
|---|---|---|---|---|---|---|---|---|
| | amount of rice stuck to tester | A6 | E30 | F6 | B1 | J6 | WK9 | S13 |
| Oryza glaberrima | 0.27 | + | + | - | + | + | + | + |
| WAB9611 | 0.57 | + | + | - | + | - | - | + |
| WITA7 | 0.50 | - | - | - | + | - | - | - |
| Cisdane | 0.41 | - | - | - | + | - | - | - |
| WAB450106 | 0.54 | - | + | - | + | + | - | + |
| +: Discrimination band appeared in electrophoresis after PCR. -: Discrimination band did not appear in electrophoresis after PCR. Physical data: in terms of the amount (L3, mm) of rice stuck to Tensipresser. | | | | | | | | |

The results confirm that the multivariate analysis in the invention in which the explanatory variables are from the digitization of the presence or absence of the discrimination DNA bands in PCR with STS primers is applicable to different varieties of rice harvested in various countries in the world for predicting the physical properties of different types of boiled rice.

Background Example 7 (Evaluation of Palatability of Polished Rice through RAPD method) :

"Koshihikari", "Hitomebore", "Akitakomachi", "Sasanishiki", "Nipponbare", "Hinohikari", "Kirara 397", "Mutsuhomare", "Kinuhikari", "Asanohikari" and "Haenuki" were sampled, and separately ground into polished rice powder in the same manner as in Example 1. A genome DNA was extracted from 6 g of each rice powder sample processed through CTAB method also in the same manner as in Example 1.

Concretely, 6 ml of a 2 % CTAB solution (0.1 M tris-HCl, 2 mM disodium ethylenediaminetetraacetate (EDTA), 1.4 M NaCl, pH 8.0) at 70°C was added to the sample and stirred, and put into an incubator at 55°C, and 6 ml of the same CTAB (1 %) solution was added thereto. In that condition, the genome DNA was extracted from the sample for 30 minutes.

Next, chloroform/isoamyl alcohol (24/1) was added to the DNA extract, stirred and centrifuged. A DNA precipitant (1 % CTAB solution, 20 mM tris-HCl, 10 mM EDTA, pH 8.0) was added to the resulting supernatant, and left at 4°C overnight to precipitate the DNA. Next, this was centrifuged, and the resulting DNA precipitate was extracted with 1 M NaCl. The DNA extract was washed with isopropyl alcohol and ethanol and then precipitated in an ordinary manner.

The DNA precipitate was dissolved in 200 $\mu$l of a TE buffer (10 mM tris-HCl, 1 mM EDTA, pH 8.0) to prepare a DNA sample solution.

The thus-extracted DNA template was subj ected to PCR with four random primers, OPB1, OPB18, OPM11 and OPT16 (all by Operon). The PCR composition was prepared by mixing 0.2 $\mu$l of Taq polymerase (by Takara Bio Inc., 5 U/$\mu$l), 2.5 $\mu$l of PCR buffer (12 mM tris-HCl, 60 mM KCl, pH 8.3), 2.0 $\mu$l of MgCl$_2$, 1 $\mu$l of the template DNA (200 ng/l $\mu$l) and 1 $\mu$l of dNTPs (100 $\mu$M). This was mixed with the random primers of 2.0 $\mu$l each, along with sterilized water to be 25.0 $\mu$l in total.

The reactor is PCR Thermal Cycler MP (by Takara Bio Inc.). In the reactor, the template DNA was subjected to 35-cycle PCR. One PCR cycle comprises denaturation at 94°C for 1 minute, annealing at 36°C for 1 minute and chain-extension at 72°C for 2 minutes.

The presence or absence of discrimination DNA bands in the band pattern of the DNA of each sample amplified through PCR were binarized. Concretely, the presence of the discrimination DNA bands (+) is 1, and the absence thereof (-) is 0.

In addition, the data thus obtained herein were processed through multiple regression analysis in which the binarized data indicating the presence or absence (1 or 0) of the discrimination bands with four random primers are the explanatory variables and the data of palatability evaluated through organoleptic examination (Table 2) are the response variables. This gave the following physical data estimation formula (palatability estimation formula) (7). The multiple correlation function derived from this physical data estimation formula for the rice samples tested is 0.89.

$$y = 5.71 + 1.04 \times OPB18 - 1.17 \times OPB1 + 2.29 \times OPM11 + 1.17 \times OPT16 \qquad (7)$$

Example 8 (Selection of Palatable Rice from Half Grains of Unpolished Rice) :

Five grains of palatable rice "Koshihikari" and 5 grains of disease-resistant non-palatable rice "Tsukinohikari" were mixed, totaling 10. Each grain was halved with a cutter, one containing its embryo and the other not.

The embryo-free half grains were individually ground in a mortar, and its DNA was extracted through treatment with CTAB method in the same manner as in Example 2. In this, however, the scale of the experiment is 1/500 of that in Example 2. The thus-obtained 10 DNAs were separately subjected to PCR for amplifying the template DNA.

The PCR composition was prepared by mixing 0.2 $\mu$l of Taq polymerase (by Takara Bio Inc., 5 U/$\mu$l), 2.5 $\mu$l of PCR buffer (12 mM tris-HCl, 60 mM KCl, pH 8.3), 2.0 $\mu$l of MgCl$_2$, 1 $\mu$l of the template DNA (200 ng/l $\mu$l) and 1 $\mu$l of dNTPs (100 $\mu$M). The STS primers for PCR are A6F21 and A6R22 (SEQ ID Nos. 3 and 4) of 0.4 $\mu$l each; A7F22 and A7R17 (SEQ ID Nos. 15 and 16) of 0.5 $\mu$l each; M11F20 and M11R20 (SEQ ID Nos. 59 and 60) of 0.6 $\mu$l each; S13F25 and S13R24 (SEQ ID Nos. 75 and 76) of 0.5 $\mu$l each; T16F24 and T16R26 (SEQ ID Nos. 83 and 84) of 0.4 $\mu$l each; J6F18 and J6R20 (SEQ ID Nos. 51 and 52) of 0.4 $\mu$l each; and WK9F20 and WK9R20 (SEQ ID Nos. 87 and 88) of 0.4 $\mu$l each, and these were mixed with the PCR composition along with sterilized water to be 25.0 $\mu$l in total.

The reactor is PCR Thermal Cycler MP (by Takara Bio Inc.). In the reactor, the template DNA was subjected to 35-cycle PCR. One PCR cycle comprises denaturation at 94°C for 1 minute, annealing at 62°C for 1 minute and chain-extension at 72°C for 2 minutes.

The amplified DNAs were then subj ected to electrophoresis in Mupid II (by Cosmobio), in which they were allowed to migrate in 2 % agarose gel for 40 minutes, and stained with ethidium bromide to give a band pattern through exposure to UV light. The results are given in Table 8.

In the same manner as in Example 1, the palatability of each sample was digitized according to the method in Rice *Palatability Ranking* (published by the Japan Association of Grain Inspection, 2001) and the method in *Encyclopedia of Rice Varieties 2* (published by Rice Data Bank in 1999). Briefly, the data digitized according to these methods were summed up, and the sum total of the data indicates the palatability of each sample. In addition, the presence or absence of discrimination DNA bands in the band pattern of the DNA of each sample amplified through PCR as in Table 8 were binarized, like in Example 1.

Based on the data shown in Table 8, the palatability estimation formula (1) in Example 1 was applied to the samples tested herein to calculate the estimated palatability value of each sample. From the thus-estimated palatability values thereof, the samples were grouped into two, group A of high values and group B of low values.

Thus grouped into two each comprised of five embryo (germ)-having half grains, the samples were processed with a 1 % sodium hypochlorite solution to sterilize their surfaces, then put on a medium having a composition mentioned below, and allowed to germinate at 30°C for 7 days. The germinated half grains were transferred into a phytotron, Biotron NC350 (By Nippon Medical Instruments Manufacturing), and cultured therein in a cycle at 28°C (in light) for 10 hours and 28°C (in dark) for 14 hours to thereby allow them to grow into green seedlings.

Next, the seedlings were transplanted into an agricultural pot containing the same medium as above, allowed to grow therein for 7 days, then transplanted into seedling compost and allowed to further grow in a phytotron, TGH-6 (by Tabai Espec) for 2 weeks. Thus having grown, the seedlings were then transplanted into a Wagner pot filled with potting compost, and cultivated in the phytotron for 4 months to thereby allow them to put forth ears.

Medium Composition (in one liter, pH 5.8):

| | |
|---|---|
| Agarose | 8.000 g |
| Ammonium sulfate | 0.463 g |
| Potassium nitrate | 2.830 g |
| Calcium chloride | 0.166 g |
| Magnesium sulfate | 0.185 g |
| Potassium dihydrogenphosphate | 0.400 g |
| Ferric sulfate | 0.278 g |
| Disodium EDTA | 0.373 g |
| Nicotinic acid | 0.250 mg |
| Vitamin B6 | 0.250 mg |

(continued)

| | |
|---|---|
| Vitamin B1 | 0.500 mg |
| Glycine | 1.000 mg |

These rice plants were further cultivated in the phytotron for 40 days to ripen their ears. Then, the ripened ears were harvested, threshed and hulled. The hulled grains were polished and ground into powder, from.which the DNAs were extracted with CTAB method, like in Example 2. The DNAs were then subjected to PCR in the same manner as above.

The amplified DNAs in the group A gave the same discrimination band pattern as that of "Koshihikari", and the amplified DNAs in the group B gave the same discrimination band pattern as that of "Tsukinohikari". After boiled, the rice samples thus identified as "Koshihikari" tasted good, but those identified as "Tsukinohikari" not good.

The process demonstrated herein supports the invention that makes it possible to select palatable rice by extracting a DNA from an embryo (germ)-free half grain of unpolished rice, processing the template DNA in PCR with STS primers to estimate the palatability of the rice, and germinating the remaining, embryo (germ)-having half grain.

Table 8 - Identification of "Koshihikari" and "Tsukinohikari" through PCR.

| Group | Estimated Value of Palatability | Primers Used, and Presence or Absence of Discrimination Bands | | | | | | | Identification of Cultivated Rice Samples by PCR |
|---|---|---|---|---|---|---|---|---|---|
| | | J6 | WK9 | A6 | A7 | M11 | S13 | T16 | |
| A | 8.809 | + | - | - | + | + | - | - | Koshihikari |
| B | 4.370 | + | + | - | - | - | - | + | Tsukinohikari |
| +: Discrimination band appeared in electrophoresis after PCR. -: Discrimination band did not appear in electrophoresis after PCR. | | | | | | | | | |

Example 9 (Selection of Palatable Rice through Hybridization Breeding and PCR of Half Grains of Unpolished Rice):

A maternal variety of palatable rice, "Koshihikari" was hybridized with a paternal variety of disease-resistant non-palatable rice "Tsukinohikari". Briefly, grains of "Koshihikari" were castrated by dipping them in warm water in an ordinary manner, and pollinated with "Tsukinohikari". Thus hybridized, the grains were cultivated, and they produced F1 seeds. Unhulled, the F1 seeds were sown in a Wagner pot filled with seedling compost and cultivated in a phytotron, TGH-6 (by Tabai Espec) through self-pollination, and they produced F2 seeds.

The F2 seeds were hulled. Each of some unpolished grains thus obtained was halved with a cutter into an embryo (germ) -free half grain and another half grain having an embryo (germ). The embryo-free half grain was ground in a mortar and processed with CTAB method to extract its DNA, like in Example 2. In this, however, the scale of the experiment is 1/500 of that in Example 2. The thus-obtained DNAs were subjected to PCR with STS primers for amplifying the template DNA. The PCR condition is the same as in Example 7.

The discrimination DNA band patterns thus obtained through PCR of the samples were applied to the palatability estimation formula (1) in Example 1 to calculate the estimated palatability values of the samples. Thus analyzed, the samples were grouped into two, one comprised of those having the thus-estimated palatability value of at most 5, and the other comprised of those having the thus-estimated palatability value of at least 8. Of the two groups, the embryo-having half grains were germinated and grown, the resulting seedlings were allowed to put forth ears, and the ears were ripened, harvested, threshed and hulled, like in Example 7. The hulled grains were polished in a laboratory rice mill, and boiled in an electric rice cooker, RC-183 (by Toshiba), and the boiled rice was organoleptically tested, like in Example 2.

The organoleptic test confirmed that the samples of the group having the estimated palatability value of at most 5 are not palatable and those of the other group having the estimated palatability value of at least 8 are palatable.

The process demonstrated herein supports the invention that makes it possible to select palatable rice by cutting each unhulled or unpolished grain of hybridized rice seeds into two, extracting a DNA from each of the embryo - free halves of unhulled or unpolished rice grains, processing the template DNA in PCR with at least two STS primer pairs to estimate the palatability of the rice, and germinating the remaining, embryo-having half grains.

**Claims**

1. A DNA-level rice palatability evaluation method for selecting palatable rice, which comprises

 • amplifying the DNA extracted from a rice plant, unhulled rice; unpolished rice, polished rice, boiled rice, rice cake or ground powder thereof through PCR in the presence of at least two STS primer pairs selected from a group of primer pairs A6F21 (SEQ ID No. 3) and A6R22 (SEQ ID No. 4); 81F25 (SEQ ID No. 11) and B1R20 (SEQ ID No. 12); A7F22 (SEQ ID No. 15) and A7R17 (SEQ ID No. 16); 843F17 (SEQ ID No. 23) and B43R18 (SEQ ID No. 24); E30F28 (SEQ ID No. 31) and E30R24 (SEQ ID No. 32); F6F25 (SEQ ID No. 35) and F6R22 (SEQ ID No. 36); G4F18 (SEQ ID No. 39) and G4R24 (SEQ ID No. 40); G22F27 (SEQ ID No. 43) and G22R23 (SEQ ID No. 44); G28F1 7(SEQ ID No. 47) and G28R28 (SEQ ID No. 48); J6F18 (SEQ ID. No. 51) and J6R20 (SEQ ID No. 52); M2CGF16 (SEQ ID. No. 55) and M2CGR15 (SEQ ID No. 56); M11F20 (SEQ ID. No. 59) and M11R20 (SEQ ID No. 60); P5F20 (SEQ ID. No. 67) and P5R25 (SEQ ID No. 68); S13F25 (SEQ ID. No. 75) and S13R24 (SEQ ID No. 76); T16F24 (SEQ ID No. 83) and T16R26 (SEQ ID No. 84); WK9F20 (SEQ ID. No. 87) and WK9R20 (SEQ ID No. 88),
 • thus obtaining various palatability evaluation bands and
 • using the resulting bands as a DNA marker for palatability evaluation to select palatable rice.

2. The method as claimed in claim 1, wherein palatability of rice is directed to

• organoleptic features of boiled rice, namely total evaluation, hardness, stickiness, appearance, palatability and aroma, and/or
• physicochemical features of rice selected from the group consisting of protein content and amylose content, and/or
• gelatinization of rice powder, and/or
• hardness and/or stickiness of boiled rice.

3. The method as claimed in claim 1 or 2, wherein the rice sample from which the DNA is extracted is an embryo-free half groin of unhulled or unpolished rice, and after the palatable rice has been selected by the use of the DNA marker obtained through PCR, the other half thereof having an embryo is allowed to germinate and grow Into a rice plant to thereby selectively breed the thus-selected variety of palatable rice.

4. The method as claimed in claim 1 , 2 or 3, wherein the PCR is effected through RAPD method using at least two STS primer pairs, digitized points of the data of a rice sample relating to the presence or absence of discrimination bonds through PCR, are used as explanatory variables, and the data thereof in any of palatability examination, organoleptic examination or physicochemical examination ore used as response variables, and these variables are processed through multivariate analysis to give a palatability estimation formula, then the multiple correlation function of each tested rice sample is calculated to determine whether said rice sample is palatable or not.

5. The method as claimed in claim 4, wherein the rice palatability evaluation in physicochemical examination is effected by measuring the properties of boiled rice.

**Patentansprüche**

1. Beurteilungsvefrahren auf DNA-Stufe für die Schmackhaftigkeit von Reis, welches umfasst

• Amplifizieren der aus einer Reispflanze, ungeschältem Reis, unpoliertem Reis, poliertem Reis, gekochtem Reis, Reiskuchen oder gemahlenem Pulver daraus extrahierten DNA durch PCR in Gegenwart von mindestens zwei STS-Primerpaaren, die ausgewählt werden aus einer Gruppe von Primerpaaren A6F21 (SEQ ID No. 3) und A6R22 (SEQ ID No. 4); B1F25 (SEQ ID No. 11) und B1R20 (SEQ ID No. 12); A7F22 (SEQ ID No. 15) und A7R1 7 (SEQ ID No. 16); B43F1 7 (SEQ ID No. 23) und B43R18 (SEQ ID No. 24); E30F28 (SEQ ID No. 31) und E30R24 (SEQ ID No. 32); F6F25 (SEQ ID No. 35) und F6R22 (SEQ ID No. 36); G4F18 (SEQ ID No. 39) und G4R24 (SEQ ID No. 40); G22F27 (SEQ ID No. 43) und G22R23 (SEQ ID No. 44); G28F17(SEQ ID No. 47) und G28R28 (SEQ ID No. 48); J6F18 (SEQ ID. No. 51) und J6R20 (SEQ ID No. 52); M2CGF16 (SEQ ID. No. 55) und M2CGR15 (SEQ ID. No. 56); M1 1 F20 (SEQ ID. No. 59) und M11R20 (SEQ ID No. 60); P5F20 (SEQ ID. No. 67) und P5R25 (SEQ ID No. 68); S13F25 (SEQ ID. No. 75) und S13R24 (SEQ ID No. 76); T16F24 (SEQ ID. No. 83) und T16R26 (SEQ ID No. 84); WK9F20 (SEQ ID. No. 87) und WK9R20 (SEQ ID No. 88),
• wodurch verschiedene Schmackhaftigkeits-Beurteilungs-Banden erhalten werden, und
• Verwendung der resultierenden Banden als DNA-Marker für die Schmackhaftigkeitsbeurteilung, um schmackhaften Reis zu selektieren.

2. Verfahren wie in Anspruch 1 beansprucht, wobei die Schmackhaftigkeit von Reis gemeint ist als

• organoleptische Merkmale von gekochtem Reis, nämlich Gesamtbeurteilung, Härte, Klebrigkeit, Aussehen, Essbarkeit und Aroma, und/oder
• physikochemische Merkmale von Reis, ausgewählt aus der aus Proteingehalt und Amylosegehalt bestehenden Gruppe, und/oder
• Gelatinisierung von Reispulver, und/oder
• Härte und/oder Klebrigkeit von gekochtem Reis.

3. Verfahren wie in Anspruch 1 oder 2 beansprucht, wobei die Reisprobe, aus der die DNA extrahiert wird, ein embryonenfreies halbes Reiskorn von ungeschältem oder unpoliertem Reis ist, und man, nachdem der schmackhafte Reis durch Verwendung des durch PCR erhaltenen Markers selektiert wurde, die andere Hälfte davon, welche einen Embryo aufweist, keimen und zu einer Reispflanze wachsen lässt, wobei **dadurch** selektiv die so selektierte schmackhafte Reissorte gezüchtet wird.

4. Verfahren wie in Anspruch 1 , 2 oder 3 beansprucht, wobei die PCR durch das RAPD-Verfahren unter Verwendung von mindestens zwei STS-Primerpaaren durchgeführt wird, digitalisierte Punkte der Daten einer Reisprobe, die sich auf das Vorhandensein oder Nichtvorhandensein von Diskriminierungsbanden durch PCR beziehen, als erklärende Variablen verwendet werden, und die Daten davon in einer Schmackhaftigkeitsuntersuchung, organoleptischen Untersuchung oder physikochemischen Untersuchung als Zielvariablen verwendet werden, und diese Variablen durch multivariate Analyse verarbeitet werden, um eine Schmackhaftigkeitsbeurteilungsformel zu erstellen, und dann die multiple Korrelationsfunktion jeder getesteten Reisprobe berechnet wird, um zu bestimmen, ob diese Reisprobe schmackhaft ist oder nicht.

**5.** Verfahren wie in Anspruch 4 beansprucht, wobei die Schmackhaftigkeitsbeurteilung von Reis in der physikochemischen Untersuchung durch Messung der Eigenschaften von gekochtem Reis durchgeführt wird.

## Revendications

**1.** Méthode d'évaluation de la sapidité du riz selon le niveau d'ADN en vue de la sélection de riz présentant un caractère de sapidité, ladite méthode comprenant les étapes consistant à :

• amplifier l'ADN extrait d'un plant de riz, de riz non décortiqué, de riz non poli, de riz poli, de riz bouilli, de gâteau de riz ou de poudre broyée de celui-ci par PRC en présence d'au moins deux paires d'amorces STS sélectionnées à partir d'un groupe de paires d'amorces A6F21 (SEQ. ID No. 3) et A6R22 (SEQ ID No. 4) ; B1F25 (SEQ ID No. 11) et B1R20 (SEQ ID No.12) ; A7F22 (SEQ ID No. 15) et A7R17 (SEQ ID No. 16) ; B43F17 (SEQ ID No. 23) et B43R18 (SEQ ID No. 24) ; E30F28 (SEQ ID No. 31) et E30R24 (SEQ ID No. 32) ; F6F25 (SEQ ID No. 35) et F6R22 (SEQ ID No. 36) ; G4F18 (SEQ ID No. 39) et G4R24 (SEQ ID No. 40) ; G22F27 (SEQ ID No. 43) et G22R23 (SEQ ID No. 44) ; G28F17 (SEQ ID No. 47) et G28R28 (SEQ ID No. 48) ; J6F18 (SEQ ID No. 51) et J6R20 (SEQ ID No. 52) ; M2CGF16 (SEQ ID No. 55) et M2CGR15 (SEQ ID No. 56) ; M11F20 (SEQ ID No. 59) et M11R20 (SEQ ID No. 60) ; P5F20 (SEQ ID No. 67) et P5R25 (SEQ ID No. 68) ; S13F25 (SEQ ID No. 75) et S 13 R24 (SEQ ID No. 76) ; T16F24 (SEQ ID No. 83) et T16R26 (SEQ ID No. 84) ; WK9F20 (SEQ ID No. 87) et WK9R20 (SEQ ID No. 88).
• ce qui permet d'obtenir diverses bandes d'évolution de la sapidité, et
• utiliser les bandes ainsi obtenues comme marqueur ADN pour évaluer la sapidité en vue de sélectionner du riz ayant un caractère de sapidité.

**2.** Méthode selon la revendication 1, dans laquelle la sapidité du riz concerne:

• les caractéristiques organoleptiques du riz bouilli , à savoir l'évaluation totale, la dureté, le caractère collant, l'aspect, la sapidité et l'arôme, et/ou
• les caractéristiques physico-chirniques du riz, sélectionnées dans le groupe constitué de la teneur en protéines et de la teneur en amylose, et/ou
• la gélatinisation de la poudre de riz, et/ou
• la dureté et/ou le caractère collant du riz bouilli.

**3.** Méthode selon la revendication 1 ou 2, dans laquelle l'échantillon de riz dont est extrait l'ADN est un demi grain de riz non décortiqué ou non poli et sans germe, et une fois que le riz ayant un caractère de sapidité a été sélectionné grâce à l'utilisation du marqueur ADN obtenu par PCR, l'autre moitié du grain de riz comprenant un germe est mis à germer et se développe pour donner un plan de riz ce qui permet de cultiver de manière sélective la variété ainsi sélectionnée de riz ayant un caractère de sapidité.

**4.** Méthode selon la revendication 1, 2 ou 3, dans laquelle la PCR est réalisée par la méthode d'amplification aléatoire de l'ADN polymorphe (RAPD) utilisant au moins deux paires d'amorces STS, des points numérisés de données d'un échantillon de riz indiquant la présence ou l'absence de bandes discriminantes par PCR, sont utilisés comme variables d'explication, et dans laquelle on utilise les données de tout examen du caractère de sapidité, de tout examen organoleptique ou de tout examen physico-chimique comme variables de réponse, ces variables étant traitées par analyse multifactorielle pour donner une formule d'évaluation du caractère de sapidité, puis on calcule la fonction de corrélation multiple de chaque échantillon de riz testé afin de déterminer si ledit échantillon de riz a un caractère de sapidité ou non.

**5.** Méthode selon la revendication 4, dans laquelle l'évaluation de la sapidité du riz par examen physico-chimique est effectuée par mesure des propriétés du riz cuit.

## FIG. 1

## FIG. 2

## FIG. 3

## FIG. 4

Multiple Correlation Function R = 0.93

(Y-axis: Found Palatability Value; X-axis: Predicted Palatability Value)

## FIG. 5

Multiple Correlation Function R = 0.87

(Y-axis: Found Palatability Value; X-axis: Predicted Palatability Value)

## FIG. 6

## FIG. 7

## *FIG. 8*

Predicted Value of Amount L3 of Boiled
Rice Grain to Stick to Tester Surface

## *FIG. 9*

Predicted Value of Physical Data H1 of Rice Samples Tested

## FIG. 10